# EUROPEAN PATENT APPLICATION

(11) **EP 2 289 552 A1**
(43) Date of publication of application: **02.03.2011**
(21) Application number: 10157170.1
(22) Date of filing: 22.05.2003
(51) Int. Cl.: A61K 39/395, A01N 63/00, A61P 41/00

(54) **An immunosuppressive agent plus anti-TCR antibodies for the induction of chimerism in transplantation**

(30) Priority: 22.05.2002 US 382680 P
(62) Divisional of application: 03738948.3
(71) Applicant: The Cleveland Clinic Foundation, Cleveland, OH 44195 (US)
(72) Inventor: Siemionov, Maria Z, Cleveland, OH 44120 (US)
(74) Representative: Weber, Martin

(57) **Abstract**

A method is provided for obtaining mixed donor-recipient chimeric cells, comprising the steps of (a) administering to an allograft recipient a short-term immunosuppressive regimen that depletes about 50% to about 99.9% of recipient T cells circulating in the peripheral blood of the recipient; (b) implanting an allograft from a donor into the recipient; (c) allowing the development of mixed donor-recipient chimeric cells in the recipient after completion of the immunosuppressive regimen; and (d) harvesting the chimeric cells from the recipient. The invention further provides method for compiling a library of chimeric cells and using the library to select of chimeric cells for the induction of tolerance to an allograft in a naïve or previously transplanted recipient.

## Description

### BACKGROUND OF THE INVENTION

Currently, 80,000 Americans are on waiting lists as organ transplant candidates. More than 3,000 new patients are added to the waiting lists each month, and about 10% of patients waiting for transplants are under the age of 18. Each day people die while awaiting a transplant of a vital organ, such as a heart, liver, kidney, pancreas, lung, bone marrow, and the like. An estimated 10,000 to 14,000 people that die each year meet the criteria for organ donation, but fewer than half become organ donors. Patients lucky enough to become transplant recipients face numerous challenges related to treatment with immunosuppressive drugs that are needed to prevent acute and chronic allograft transplant rejection.

The requirement for life-long immunosuppressive drug therapy to prevent chronic graft rejection carries a significant risk due to the nonspecific immunosuppressive effects of these drugs. Any reduction in the immune responsiveness to the allograft is accompanied by reduced immunity to infectious and malignant diseases. Therefore, the ultimate goal of transplantation surgery is induction and maintenance of donor-specific immunological tolerance without the need for lifelong immunosuppressive drug treatment regimens.

Several experimental designs to introduce new therapeutic strategies in transplantation have been reported. For example, one strategy is based on creation of stable hematopoietic chimerism within the transplant recipient. This approach requires preconditioning of the recipient to create a "space" needed for engraftment of the transplanted syngeneic and allogeneic bone marrow cells, by whole body irradiation, or other cytoablation procedures, and the like. However, the potential morbidity associated with preconditioning treatment regimens present a significant obstacle to the introduction of these strategies into use in clinical transplantation.

Therefore, it is a goal of allogeneic transplantation to provide effective, non-toxic treatments that can ensure indefinite graft survival. In particular, there is a need for the reliable induction and maintenance in the recipient of immunological tolerance to donor-specific antigens without the need for chronic immunosuppressive regimens or recipient preconditioning.

### SUMMARY OF THE INVENTION

The invention provides a novel approach to inducing and maintaining tolerance to allografts without chronic immunosuppressive regimens, without the occurrence of graft versus host disease (GVHD) and, preferably, without the necessity for recipient preconditioning. This new approach is based on our unexpected discovery that long-term allograft tolerance after a short course of immunosuppressive therapy is associated with the development of a stable hematopoietic mixed donor-recipient chimerism in the recipient without recipient preconditioning. This discovery is disclosed in our copending, co-owned U.S. Patent Application, Serial No. 10/427,013, filed April 30, 2003, entitled, "Induction and Maintenance of Tolerance to Composite Tissue Allografts" and in our copending, co-owned U.S. Patent Application, filed May 22, 2003, entitled "Chimeric Allograft Tolerance Induction, Monitoring And Maintenance," the entire disclosures of both of which are hereby incorporated by reference.

As used herein, mixed donor-recipient chimerism is used to described a state in which tissue or cells from a donor are able to live and function within a recipient host without rejection or the occurrence of GVHD. In a semi-allogeneic transplantation, where the donor and the recipient share at least one major histocompatibility complex (MHC) class I or class II locus, and the chimeric cells exhibit cell surface histocompatibility antigens of both the donor and the recipient (*i*.*e*., they are double positive). In a fully allogeneic transplantation, the donor and recipient do not share an MHC locus. In these chimeras, cells from the donor and cells from the recipient co-exist in the recipient, and these are both recognized as "self" and not rejected.

For purposes of this disclosure, the term "chimera" or "chimerism" is further intended to encompass trimeric and multimeric states such as, but not limited to, (a) states in which the recipient may have cells exhibiting both donor and recipient surface histocompatibility antigens, as well as cells from a third or multiple additional donors that are recognized as "self" by the recipient, all co-existing in the recipient; (b) states in which the recipient may have cells from three or multiple donors that are recognized as "self" by the chimeric recipient; and (c) all possible combinations and permutations of the foregoing, without limitation.

The methods according to the invention are fully applicable to transplantation of any type of allograft including, but not limited to, composite tissue such as, but not limited to human hand, human finger, human larynx, joints such as knee, hip, and the like; solid organs and glands such as, but not limited to, heart, lung, kidney, liver, pancreas, thyroid, and the like; glandular cells such as, but not limited to, islet cells and the like; skin; hematopoietic tissue; lymphoid tissue; tendons; ligaments; muscles; nerve tissue; vascular tissue such as vessels; and the like, without limitation.

In one embodiment, the invention provides a method for obtaining mixed donor-recipient chimeric cells, comprising the steps of (a) administering to an allograft recipient a short-term immunosuppressive regimen that depletes about 50% to about 99.9% of recipient T cells circulating in the peripheral blood of the recipient; (b) implanting an allograft from a donor into the recipient; (c) allowing the development of mixed donor-recipient chimeric cells in the recipient after completion of the immunosuppressive regimen; and (d) harvesting the chimeric cells from the recipient. Preferably, the chimeric cells are stored after harvesting, by cryogenic or other known cell storage methods. More preferably, the harvested chimeric cells are propagated in culture to expand their numbers prior to storage.

In a preferred embodiment of the invention, after harvesting, the expression of surface histocompatability antigens of the chimeric cells is determined and the chimeric cells are accordingly categorized as to histocompatibility antigen expression "type". These categorized chimeric cells can then be stored to become part of a library of similarly typed stored chimeric cells from multiple allograft transplant recipients, providing a multiplicity of histocompatability types. Preferably, the chimeric cells can be obtained from transplant recipients on a world-wide basis, ultimately providing a library or universal chimera bank containing every possible combination of histocompatability types.

The categorized chimeric cells obtained as described above can be used to induce and/or maintain immunological tolerance in a naive recipient requiring an allogeneic trransplant, for example, or in a transplant recipient requiring an additional transplant. According to this embodiment of the invention, a method is provided for inducing allograft tolerance and/or mixed donor-recipient chimerism in an allograft recipient, comprising the steps of (a) determining a histocompatibility antigen type of an allograft recipient; (b) determining a histocompatibility antigen type of an allograft donor; (c) selecting chimeric cells from a library of chimeric cells, wherein the selected chimeric cells represent a best match for the histocompatibility antigen types of both the recipient and the donor; (d) administering to an allograft recipient a short-term immunosuppressive regimen that depletes about 50% to about 99.9% of recipient T cells circulating in the peripheral blood of the recipient; (e) administering to the recipient a therapeutically effective amount of the selected chimeric cells; (f) implanting an allograft from a donor into the recipient, wherein a combination of steps (d) and (e) results in recipient immunological tolerance to the allograft.

Optionally, the method further comprises the individual optional steps of monitoring a level of circulating chimeric cells in the allograft recipient or monitoring the allograft for a visual or histological sign of rejection; readministering a therapeutically effective amount of the selected chimeric cells to the recipient if the level of circulating chimeric cells declines to a preselected minimum level or a sign of rejection is observed; and/or readministering a short-term immunosuppressive regimen if the level of circulating chimeric cells declines to a preselected minimum level or a sign of rejection is observed. In a preferred embodiment, the preselected minimum level of circulating chimeric cells is a minimum level for preventing onset of allograft rejection.

In an alternative embodiment, the selected chimeric cells can comprise chimeric cells produced as the result of fusion of cells of different histocompatibility antigen types, to produce cells having surface histocompatibility antigens of two or more of the cell types. Such manipulation can take place *ex vivo* by known cell fusion methods such as, but not limited to, electrofusion techniques or polyethylene glycol (PEG) fusion, and the like.

The invention further provides method for compiling a library of chimeric cells. Preferably chimeric cells are harvested from an allograft recipient or produced by cell fusion methods. The method includes determining expression of surface histocompatability antigens of the chimeric cells; optionally culturing the harvested chimeric cells to obtain an expanded population of the chimeric cells; and storing the chimeric cells.

The invention also provides a library of chimeric cells and an animal model of chimeric recipients produced according to the embodiments described above. The invention is particularly useful for allograft transplantation in human recipients, and also encompasses rodent and/or higher mammal recipients, and the like.

### BRIEF DESCRIPTION OF THE DRAWINGS

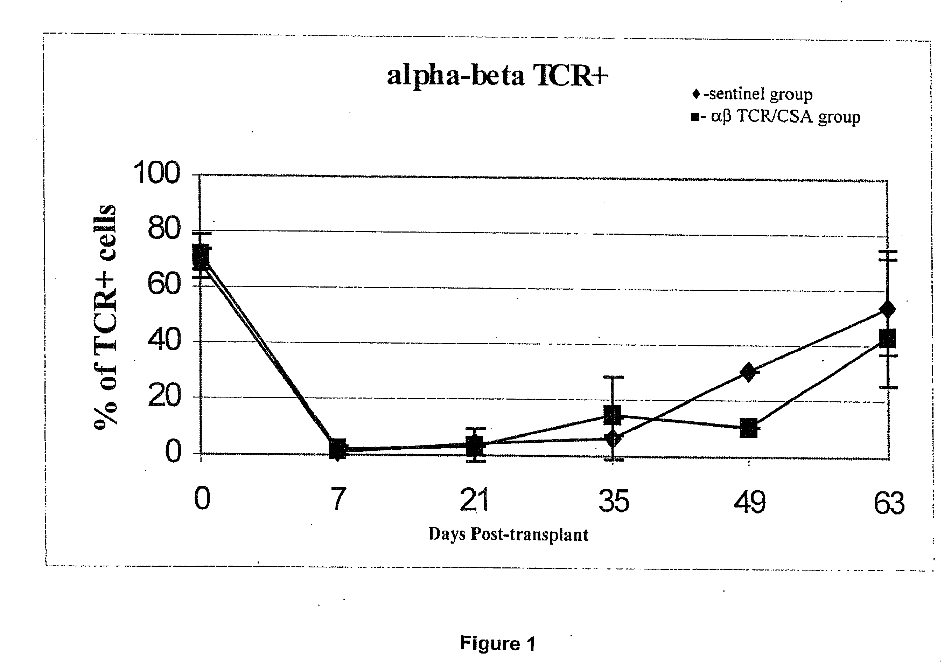

**Figure 1** illustrates flow cytometric (FC) evaluation of the peripheral blood αβ TCR⁺ T cells in recipients receiving skin and crude bone marrow semi-allogeneic transplants and treated with combined CsA/αβ TCR mAb immunosuppressive therapy. FC analysis at day 7 post-transplantation demonstrated >90% depletion of the αβ TCR⁺ T cells, with gradual reconstitution to pre-transplant levels at day 63 post-transplantation. Sentinel (untransplanted) animals treated with the combined CsA/αβ TCR mAb served as controls.

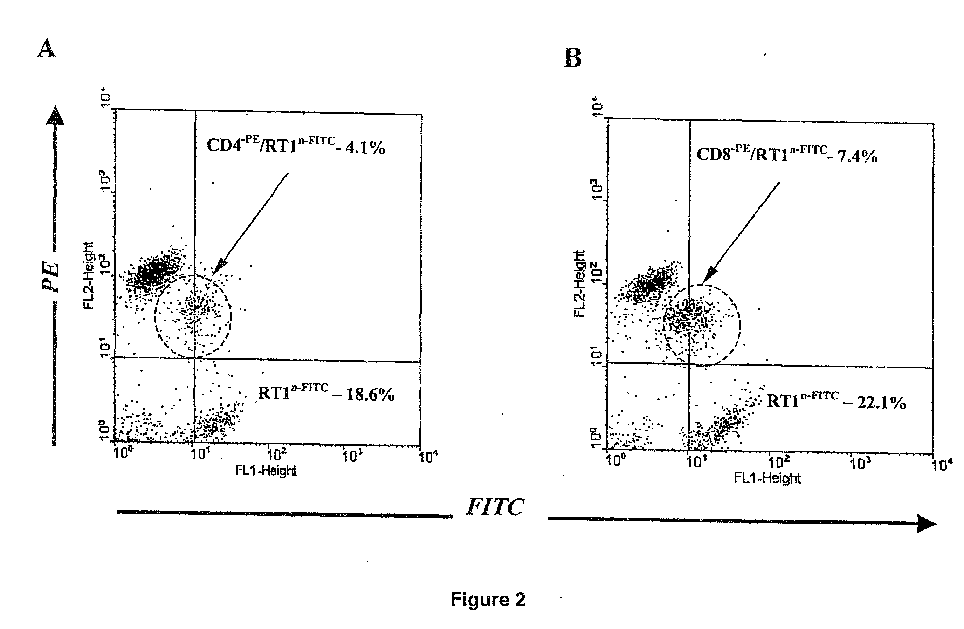

**Figure 2** illustrates FC determination of the donor-originated RT-1ⁿ expression on CD4⁺ (**A**) and CD8⁺ (B) T cell subpopulations isolated from peripheral blood of recipients of skin and crude bone marrow transplantation and treated with combined CsA/αβ TCR mAb immunosuppressive therapy. Dot-plot results of the lymphoid cell subpopulations obtained from quadrangle analytical gates at day 65 after allotransplantation demonstrated the presence of the multilineage donor-specific chimerism ranging from 18.6% to 22.1% (RT-1ⁿ positive cells). Examination of two-color stained RT-1^{n-FITC}/CD4^{-PE} and RT-1^{n-FETC}/CD8^{-PE} peripheral lymphocytes revealed 4.1% and 7.4%, respectively, of double positive CD4 and CD8 T cell subpopulations.

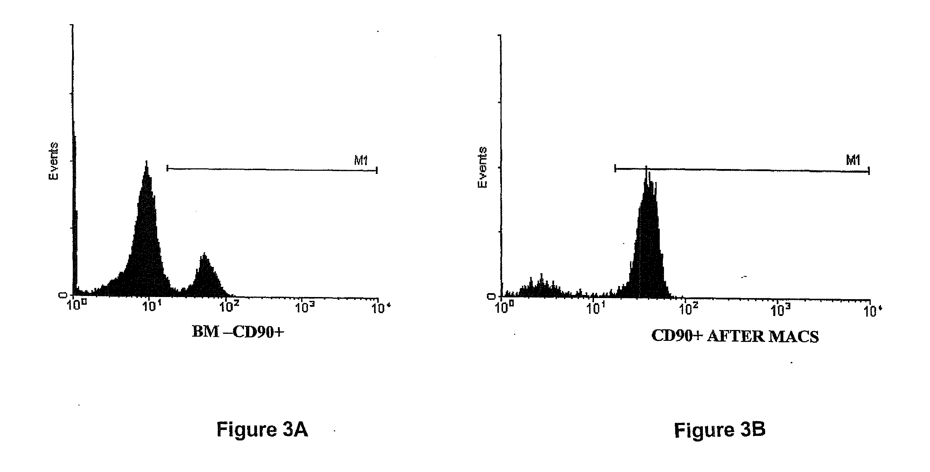

**Figure 3** illustrates FC analysis of the CD90⁺ antigen expression on the surface of stem and progenitor cells from donor bone marrow before and after selection using a magnetic MiniMACS separating system. Figure 3A shows CD90⁺ cells as the small peak, and the remainder of the bone marrow nucleated cells as the larger peak, prior to selective separation of the CD90⁺ cells from a suspension of bone marrow cells. The CD90⁺ cells comprised about 22% of the bone marrow nucleated cells. Figure 3B shows the purity of separation of the CD90⁺ cells (large peak). Less than 5% were CD90⁻. Over 95% of analyzed cells expressed the CD90 antigen, indicating high efficacy of the selection.

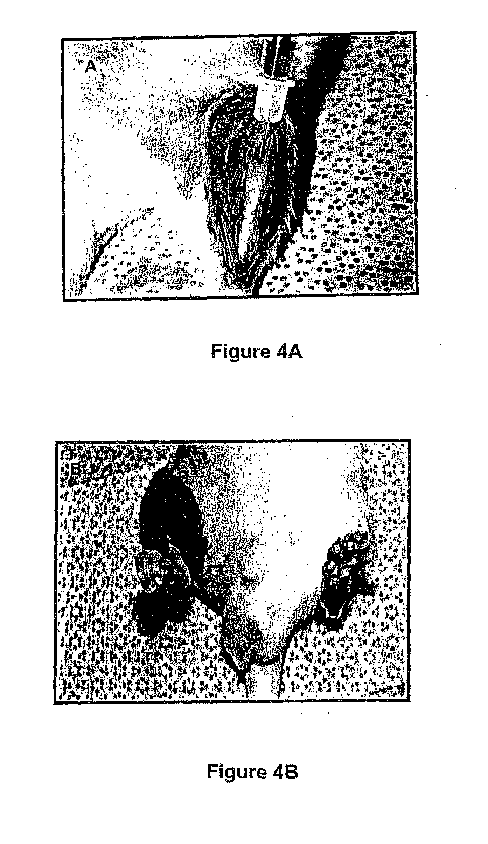

**Figure 4** illustrates intraosseous transplantation of the donor-derived stem and progenitor cells. **Figure 4A** illustrates the injection of the stem and progenitor cells directly into the bone marrow cavity of the recipient's left tibia. Following injection, the right hindlimb from the same donor was transplanted to the recipient (**Figure 4B**).

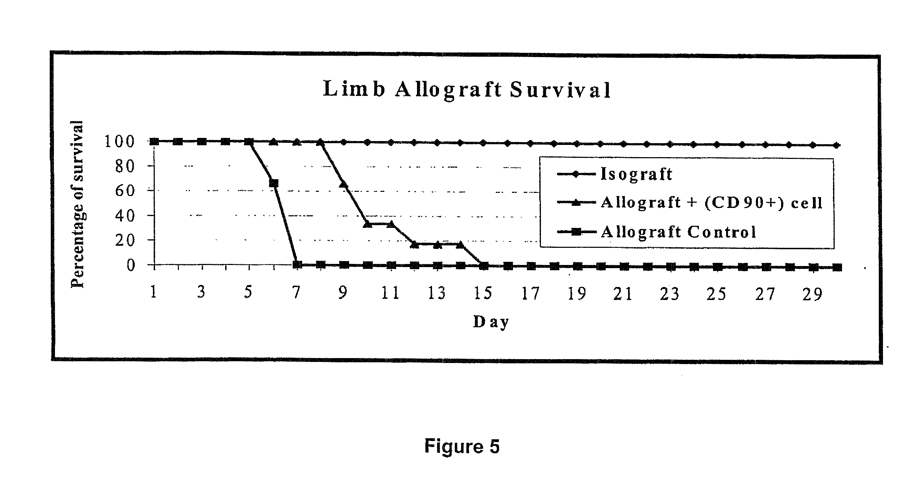

**Figure 5** illustrates a hindlimb allograft survival chart indicating significant extension of hindlimb allograft survival (p<0.05) following perioperative injection of 8-12 x 10⁵ stem and progenitor cells (CD90⁺ cells) directly into the bone marrow cavity of the hindlimb allograft recipients without an immunosuppressive protocol.

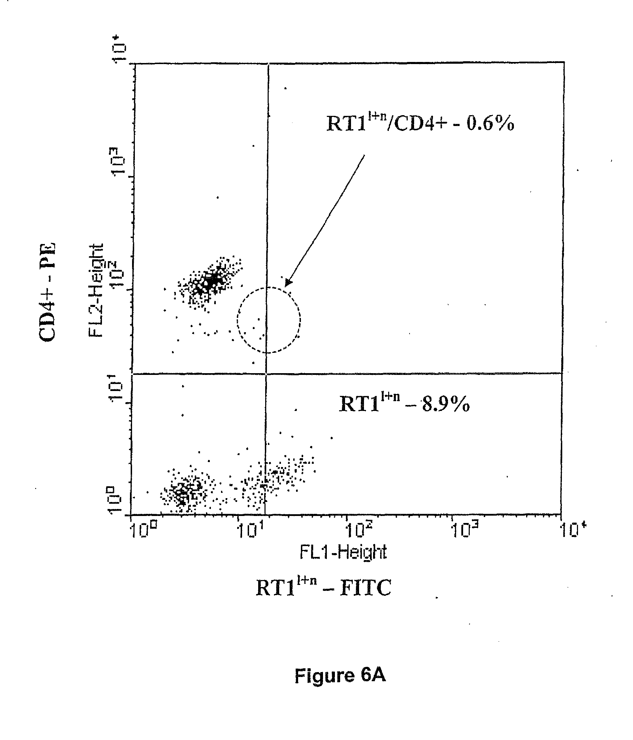

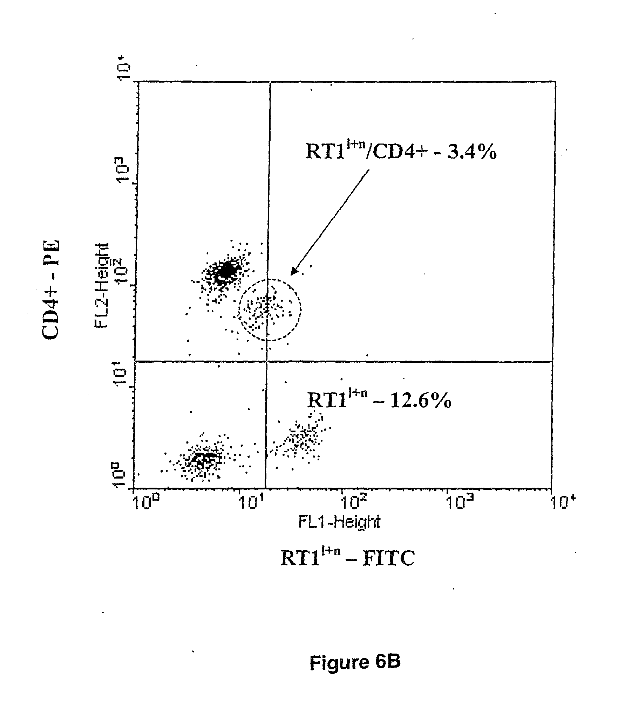

**Figure 6** illustrates a two-color flow cytometric analysis at day 14 after hindlimb allograft transplantation, showing transient chimerism in the allograft control treatment (0.6%, **A**) and a high level (3.4%, **B**) of double positive RT-1¹⁺ⁿ/CD4⁺ chimeric cells in the peripheral blood of limb recipients treated with the intraosseous injection of donor CD90⁺ stem and progenitor cells at the time of transplantation.

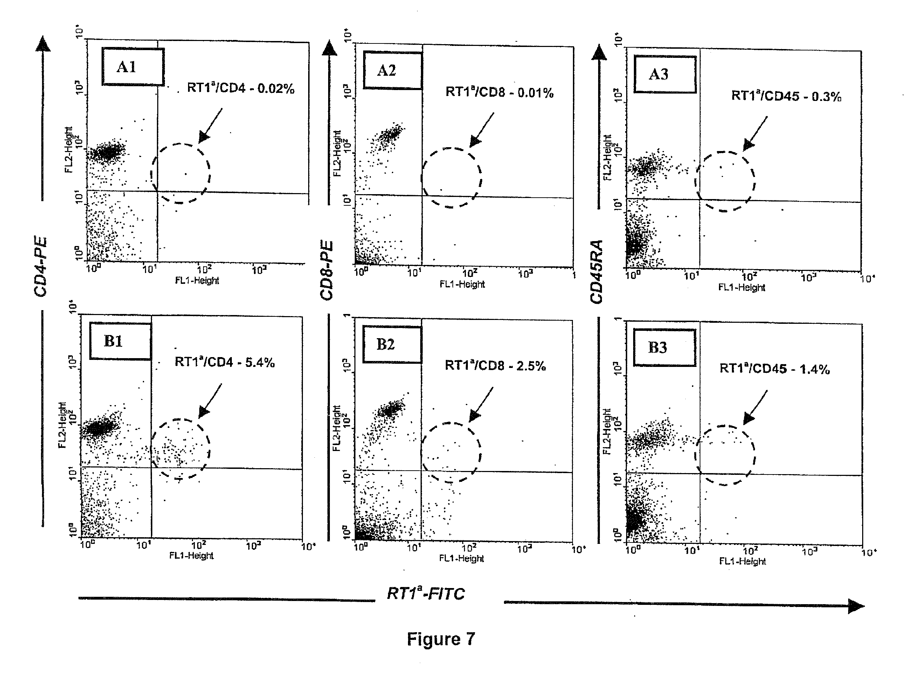

**Figure 7** illustrates flow cytometric analysis at the day 35 after hindlimb allograft transplantation, showing high levels of multilineage donor-specific lymphoid chimerism (**B1-B3**) in the peripheral blood mononuclear cells (PMBC) of the recipients receiving direct intraosseous injection of donor stem and progenitor cell. In contrast, intravenous injection of the same number of cells resulted in low-level, transient chimerism (**A1-A3**), indicating that bone creates more permissive conditions for donor stem and progenitor cell engraftment.

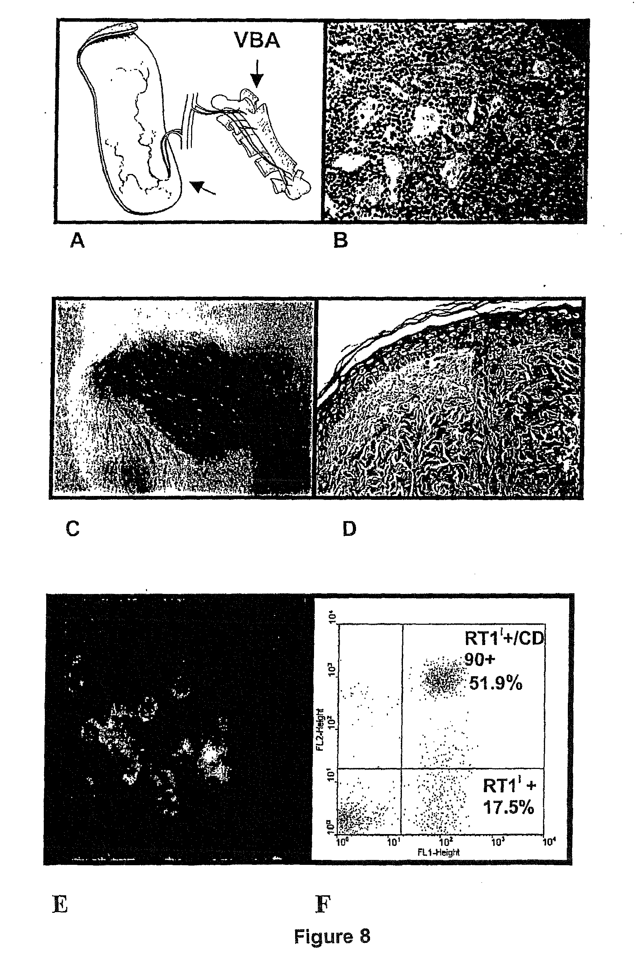

**Figure 8** illustrates vascularized skin and bone allografts (VSBA). A schematic representation of the VSBA model combining a superficial epigastric skin flap and a vascularized femoral bone allograft (**A**). **B** shows a Giemsa stained vascularized bone marrow isograft 7 days after transplantation, showing over 99% viability of the bone marrow cells in these transplants. **C** shows an accepted VSBA transplant allograft across a fully mismatched major MHC barrier (Brown Norway donor, Lewis recipient) at day 63 after cessation of immunosuppressive protocol, showing full acceptance of the vascularized skin allograft. **D** shows the skin biopsy (hematoxylin and eosin stained) with preserved dermis and epidermis and no histological signs of rejection. **E** shows the immunohistostained frozen sections of the bone marrow isolated from the donor vascularized bone allograft after transplantation into the recipient, at day 63 after cessation of immunosuppressive protocol. **F** illustrates flow cytometry analysis of isolated cells. More than 50% of the cells in the donor bone marrow were recipient CD90⁺/RT-1^{L} (related to LEW MHC class I) positive cells, showing the trafficking of recipient cells into the donor bone marrow, and also confirming the viability of the transplanted vascularized bone marrow.

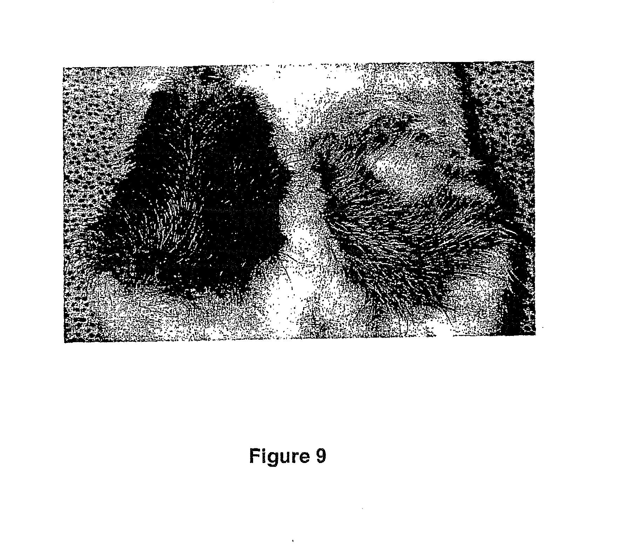

**Figure 9** illustrates a LEW recipient of two genetically unrelated VSBA transplants at day 35 after transplantation. The vascularized bone transplants are not visible, as they are beneath the transplanted skin flaps shown. On the left is a VSBA transplant from a BN donor, showing full skin acceptance by the LEW recipient of this fully allogeneic transplant. On the right is a VSBA transplant from an ACI donor, showing full skin acceptance by the LEW recipient of this fully allogeneic transplant.

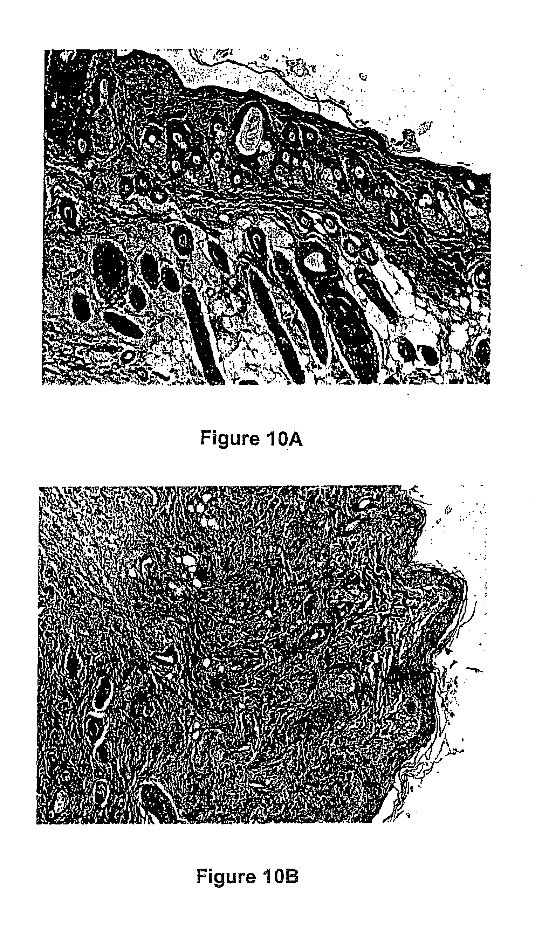

**Figures 10A** and **10B** illustrate H&E stained formalin-fxed skin tissues taken from the BN allograft and the ACI allograft, respectively, at day 21 after transplantation, showing preserved dermis and epidermis and no histological signs of rejection.

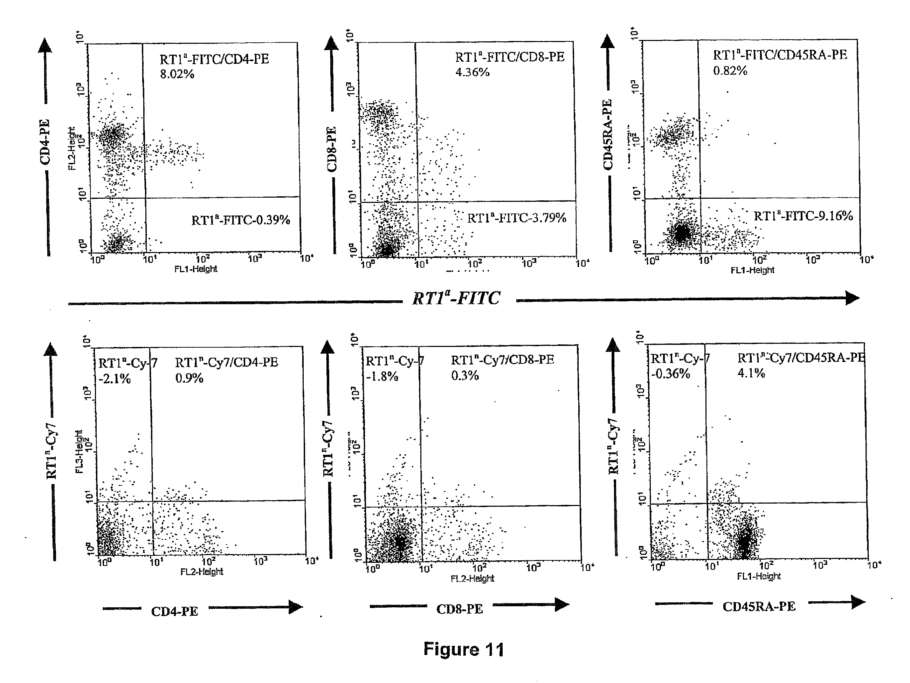

**Figure 11** illustrates flow cytometry analysis performed on PBMC of the LEW recipients at day 21 after transplantation of VSBA transplants from both the BN and the ACI donors. The dot-plot results of the lymphoid cell subpopulations obtained from quadrangle analytical gates demonstrated the presence of double positive RT-1^{a-FITC}/CD4^{-PE}, RT-1^{a-FITC}/CD8^{-PE} and RT-1^{a-FITC}/CD45RA^{-PE} (ACI/LEW) at a level of 8.02%, 4.36% and 0.82%, respectively, of PBMC ( top horizontal row); and also the presence of double positive RT-1^{n-Cy7}/CD4^{-PE}, RT-1^{n-Cy7}/CD8^{-PE} and RT-1^{n-Cy7}/CD45RA^{-PE} (BN/LEW) at a level of 0.9%, 0.3% and 4.1%, respectively (bottom horizontal row).

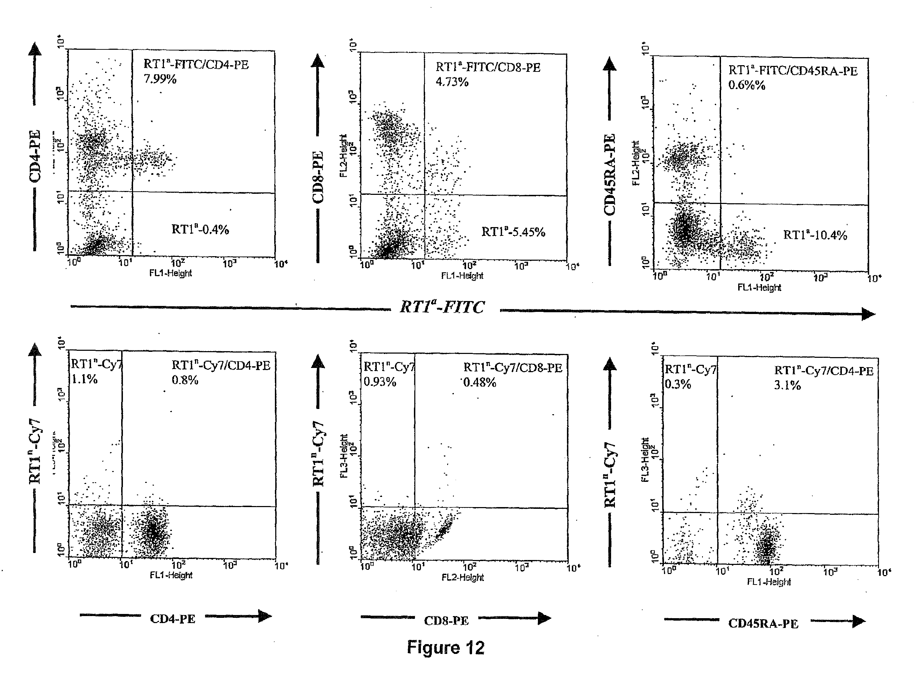

**Figure 12** illustrates flow cytometry analysis performed on PBMC of the LEW recipients at day 35 after transplantation of VSBA transplants from both the BN and the ACI donors. The dot plot results demonstrated the presence of double positive RT-1^{a-FITC}/CD4^{-PE}, RT-1^{a-FTTC}/CD8^{-RE} and RT-1^{a-FITC}/CD45RA^{-PE} (ACI/LEW) at a level of 7.99%, 4.73% and 0.6%, respectively, of PBMC (top horizontal row); and also the presence of double positive RT-1^{n-Cy7}/D4^{-PE}, RT-1^{n-Cy7}/CD8^{-PE} and RT-1^{n-Cy7}/CD45RA^{-PE} (BN/LEW) at a level of 0.8%, 0.48% and 3.1%, respectively (bottom horizontal row).

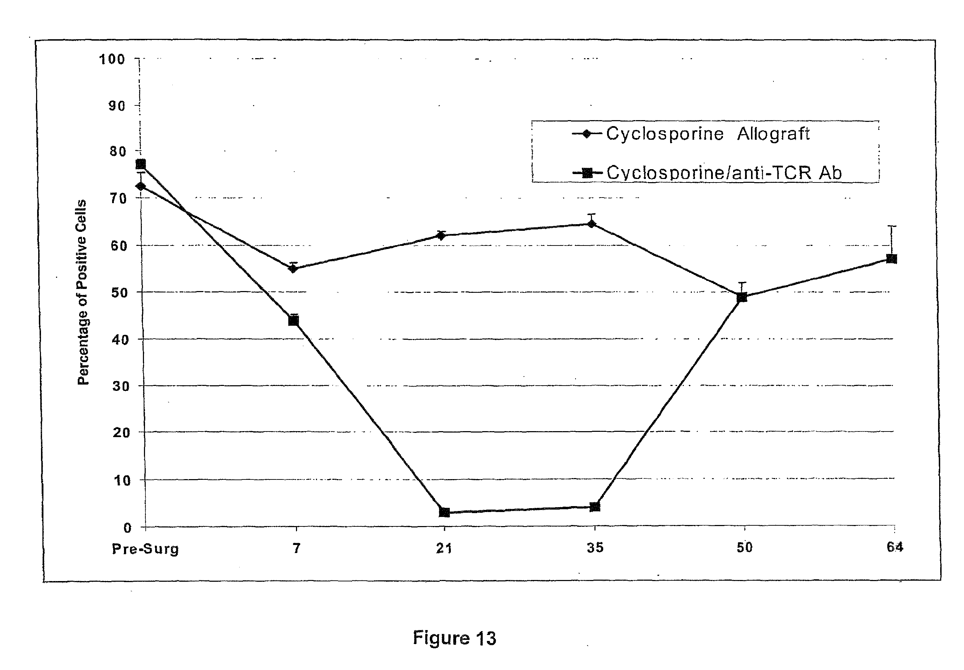

**Figure 13** illustrates a flow cytometry determination of *in vivo* immunodepletion of αβ TCR⁺ cells evaluated in the peripheral blood of the semi-allogeneic grafted animals treated with CsA alone or with the combined CsA/αβ-TCR mAb therapy in the 35-day protocol.

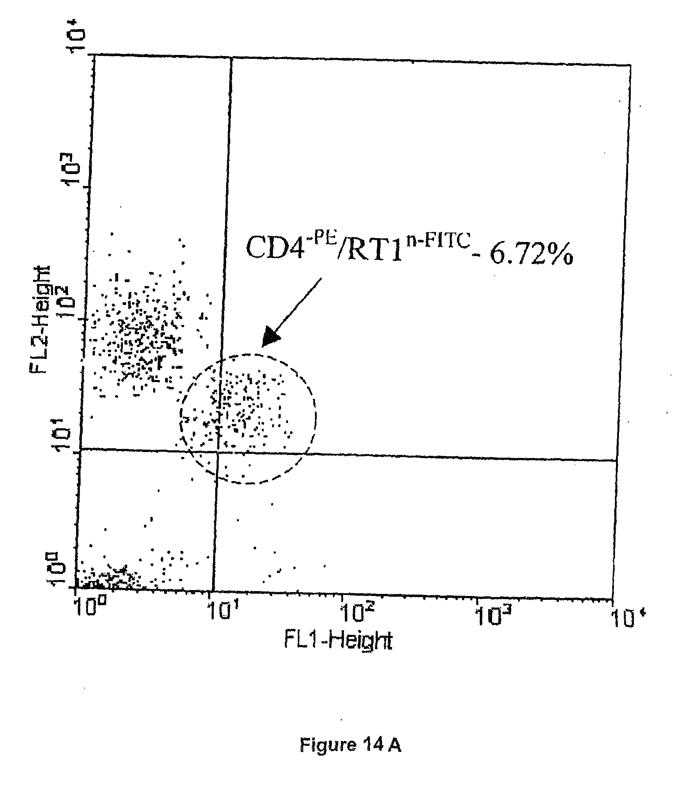

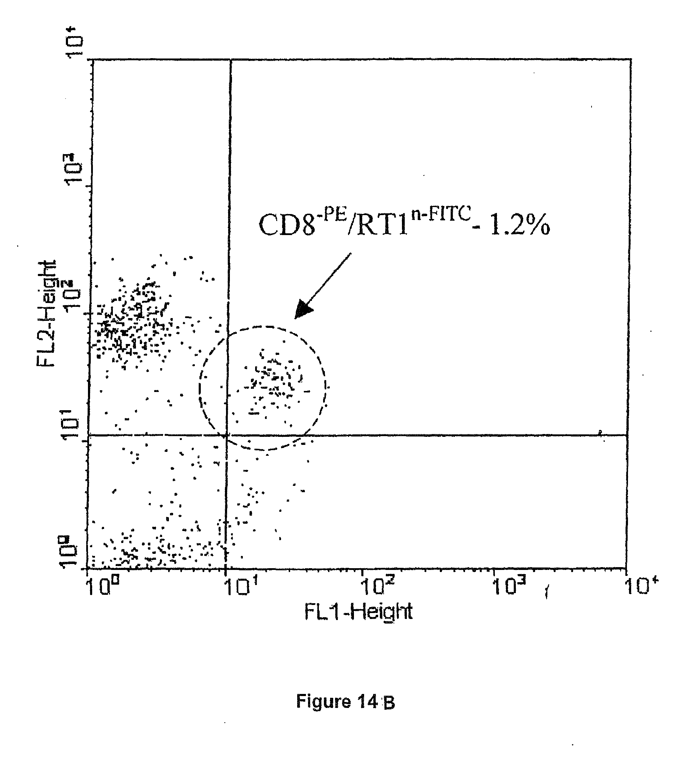

**Figures 14A** and **14B****,** respectively, illustrate lymphoid chimerism to both the donor and recipient CD4⁺ and CD8⁺ T-cell subpopulations in a representative nonmyeloablated non-conditioned recipient treated with the combined CsA/αβ-TCR mAb therapy in the 35-day protocol. The FC determination of the donor-specific chimerism revealed the presence of double positive CD4^{PE}/RT-1^{nFITC}(6.72%) and CD8^{PE}/RT-1^{nFITC} (1.2%).

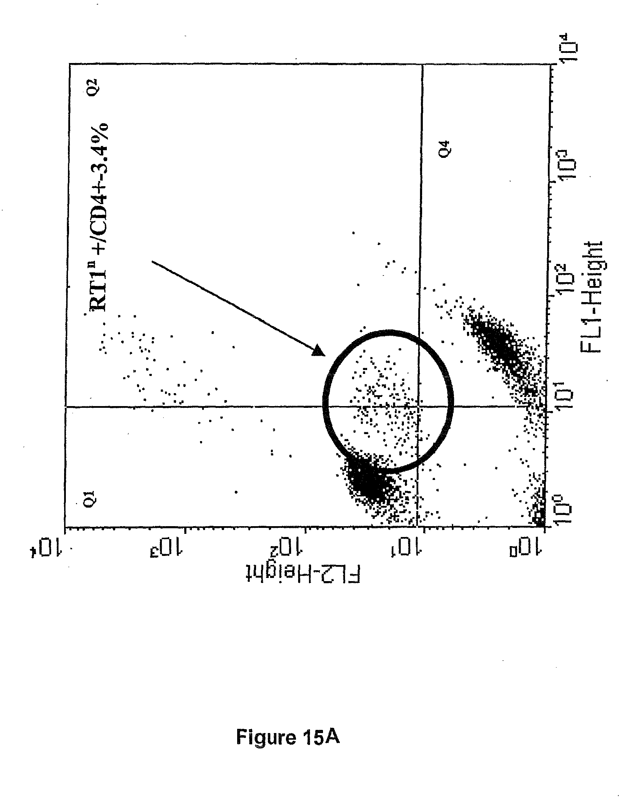

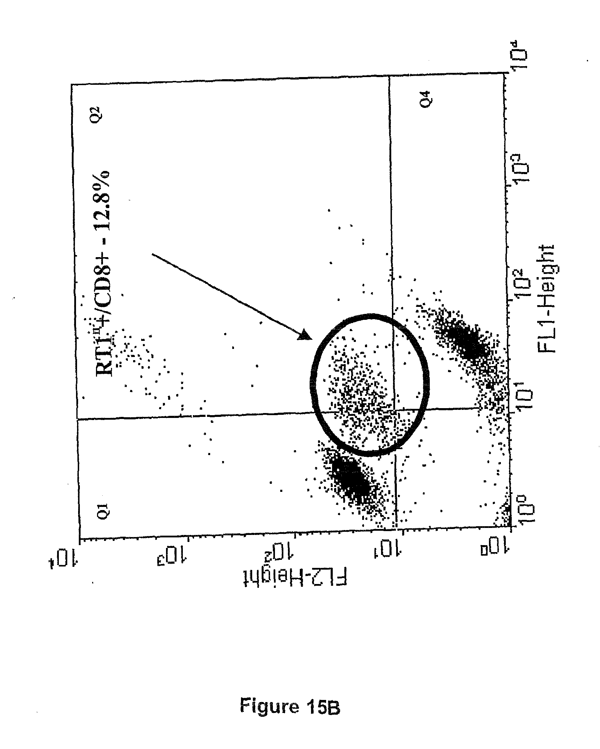

**Figures 15A** and **15B** illustrates double-positive donor CD4^{PE}IRT-1^{nFITC} (3.4%) (15A) and CD8^{PE}/RT-1^{nFITC} (12.8%) (15B) T-cell subpopulations at 150 days post-transplant in the 35-day combined CsA/αβ-TCR mAb protocol.

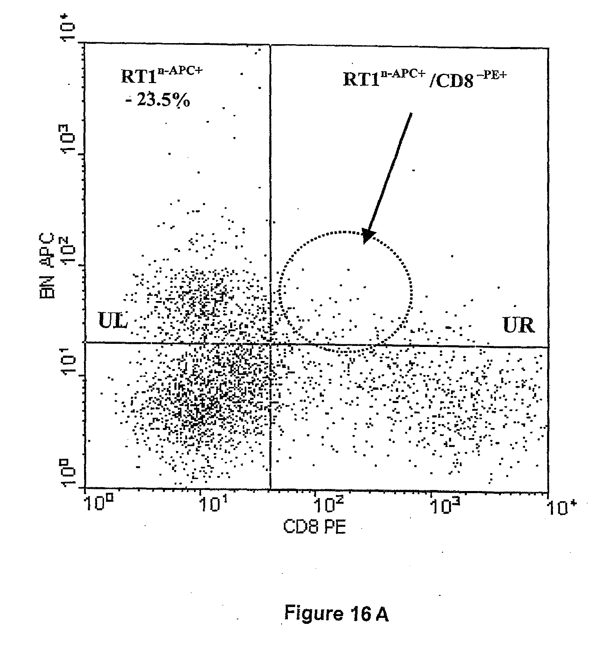

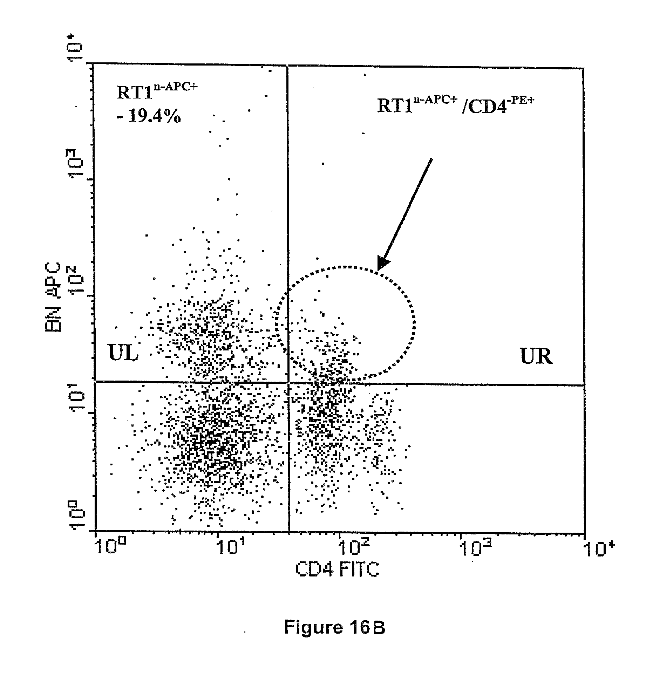

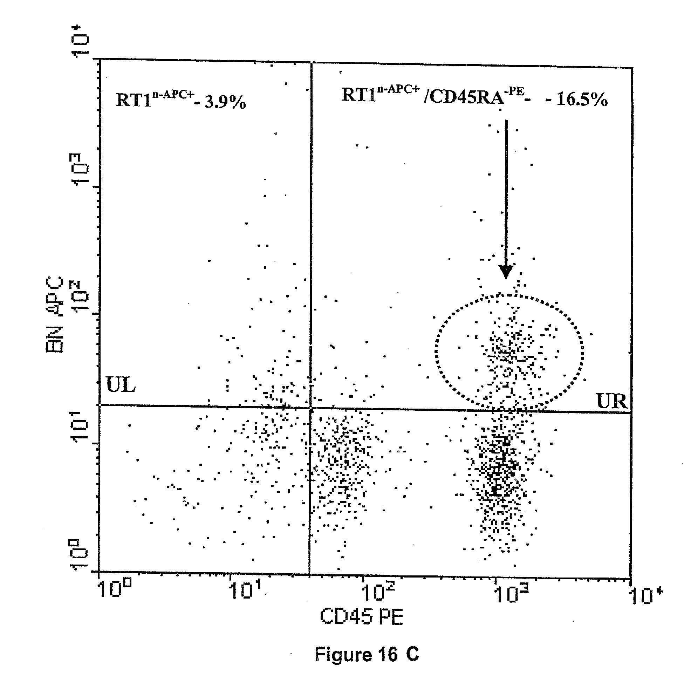

**Figures 16A****,** **16B** and **16C****,** respectively, illustrate flow cytometric triple-staining analysis of the donor-specific chimerism in long-term fully-allogeneic graft survivors of a 7-day combined CsA/αβ-TCR mAb treatment protocol at 120 days after transplantation, showing that 1.3% of recipient peripheral blood mononuclear cells (PBMC) were CD8⁺ cells of donor origin; 7.6% of the recipient PBMC were CD4⁺ cells of donor origin; and 16.5% of the recipient PBMC were CD45RA⁺ B cells of donor origin, respectively.

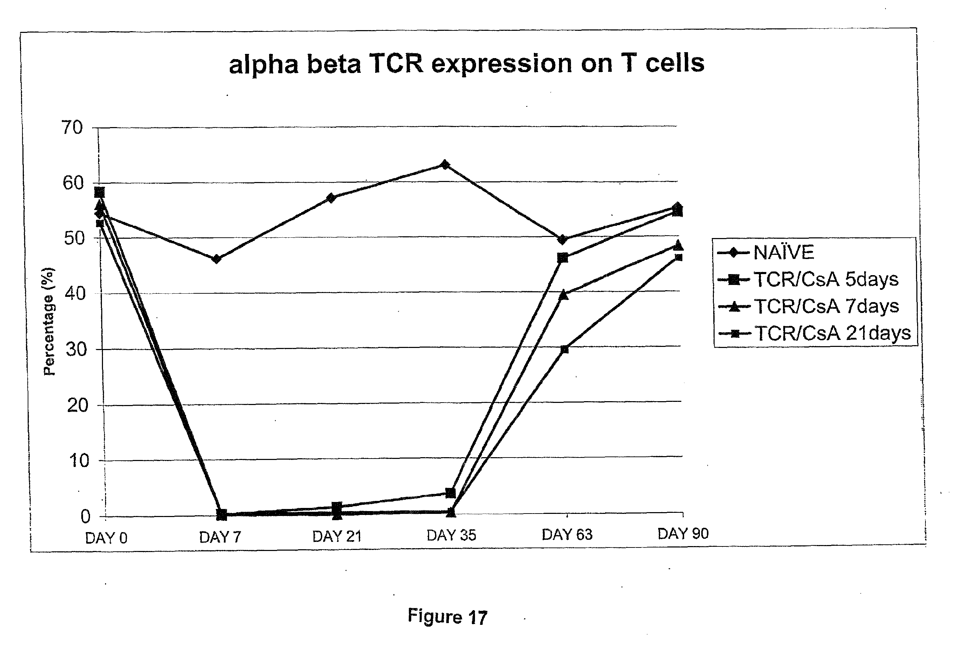

**Figure 17** illustrates a flow cytometry determination of *in vivo* immunodepletion of αβ-TCR⁺ peripheral blood T cells under the 5-day, 7-day, and 21-day protocols using the combined CsA/αβ-TCR mAb therapy.

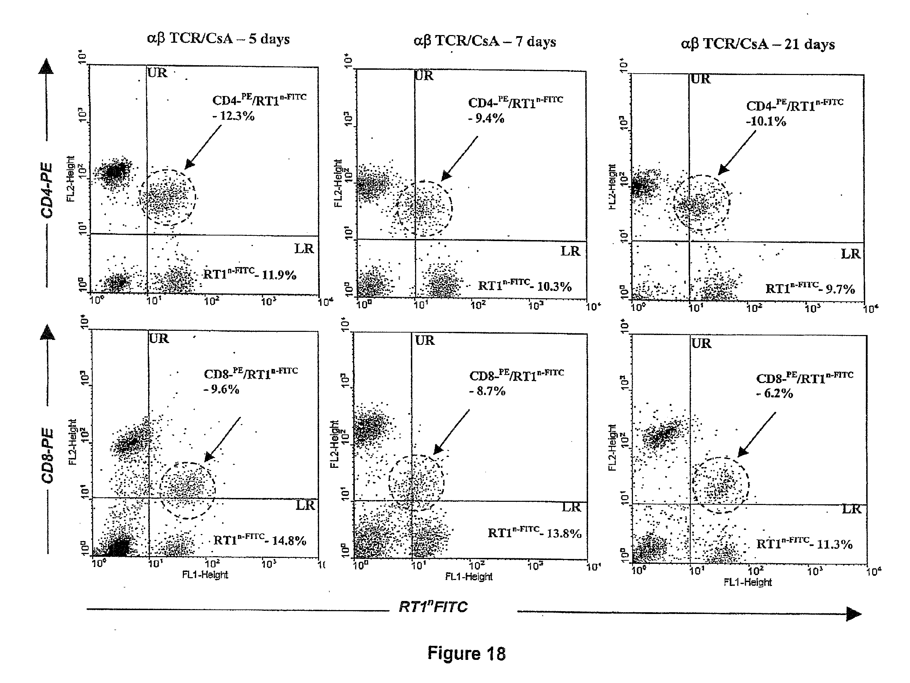

**Figure 18** illustrates peripheral blood lymphoid chimerism in fully-allogeneic recipients treated with the combined CsA/αβ-TCR mAb therapy in the (5-II), 7-day (7-II), and 21-day (21-II) protocols. The FC determination of the donor-specific chimerism revealed the presence of double positive CD4^{PE}/RT-1^{nFITC} (12.3%) and CD8^{PE}/RT-1^{nNFITC} (9.6%) cells under the 5-day protocol, double positive CD4^{PE}/RT-1^{nFITC} (9.4%) and CD8^{PE}/RT-1^{nFITC} (8.7%) cells under the 7-day protocol, and double positive CD4^{PE}/RT-1^{nFITC} (10.1%) and CD8^{PE}/RT-1^{nFITC} (6.2%) cells under the 21-day protocol.

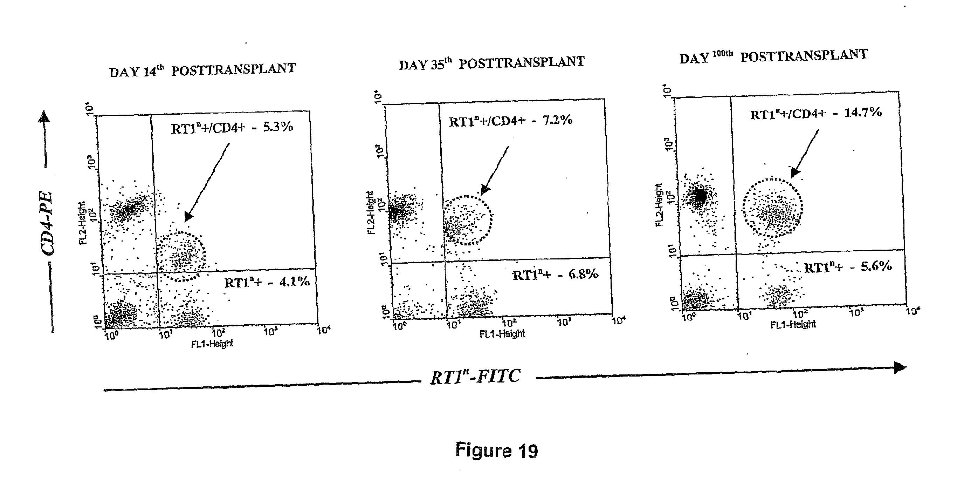

**Figure 19** illustrates the kinetics of the rise in the level of chimeric donor/recipient cells in the peripheral blood of the combined treatment allotransplant recipients leading to indefinite graft survival. Treated recipients of fully-allogeneic transplants exhibited 5.3% chimeric CD4⁺ cells by about day 14 after transplantation. This level rose to 7.2% by about day 35 after transplantation, and continued to rise to about 14.7% by about day 100 after transplantation.

### DETAILED DESCRIPTION OF THE INVENTION

The short-term immunosuppressive regimen employed in embodiments of the invention methods can include any known regimen that depletes about 50% to about 99.9% of recipient T cells circulating in the peripheral blood of the recipient. Preferably, the regimen depletes about 75% to about 95% of the circulating T cells and, more preferably, depletes about 80% to at least about 90% of the circulating T cells. The preferred immunosuppressive regimens provide significant depletion of the recipient T-cell population at the end of the immunodepleting therapy, as well as allow repopulation of the recipient T cell repertoire once the treatment protocol is withdrawn.

Exemplary suitable preferred immunosuppressive regimens are disclosed in our copending co-owned patent applications incorporated by reference above, and are here described in more detail. In particular, a preferred method for inducing mixed donor-recipient chimerism in an allograft transplant recipient comprises the steps of: administering to an allograft transplant recipient an effective amount of a pharmaceutical composition that comprises an immunosuppressive T cell-depleting agent, and an effective amount of a pharmaceutical composition that comprises anti-αβ TCR⁺ T cell receptor antibodies, wherein administration of the combination to the recipient results in elimination of about 50% to about 99.9% of T cells circulating in peripheral blood of the recipient; and implanting an allograft from an allograft donor into the recipient. A more preferred method for inducing mixed donor-recipient chimerism in allograft transplant recipient comprises an additional step; (c) implanting a therapeutically effective amount of bone marrow cells from the allograft donor into the allograft recipient.

Implanting a therapeutically effective amount of the bone marrow cells from the allograft can preferably be achieved by a delivery system such as, but not limited to, an implantable vascularizable bone from the allograft donor including the donor bone marrow; implantable crude donor bone marrow; an implantable donor bone marrow nucleated cell suspension; an implantable isolated subpopulation of donor bone marrow cells; and the like, and combinations of the foregoing.

Preferably, the immunosuppressive agent and the anti-αβ T cell receptor antibodies are administered according to a protocol such as those disclosed and claimed in the incorporated patent applications. The immunosuppressive agent and the anti-αβ T cell receptor antibodies are preferably administered in an amount, at a frequency, and for a duration of time sufficient to induce mixed donor-recipient chimerism in the allograft recipient.

In some embodiments, the immunosuppressive T cell deleting agent and the anti-αβ TCR⁺ T cell receptor antibodies are administered as a pharmaceutical composition that comprises both the immunosuppressive agent and the antibodies.

The immunosuppressive agent is preferably an inhibitor of the calcineurin pathway of T cell activation such as, but not limited to, cyclosporine A (CsA), FK-506, and the like; or other inhibitors of IL-2 production such as, but not limited to, rapamycin and the like, and combinations of the foregoing. More preferably, the immunosuppressive agent is CsA.

The anti-αβ T cell receptor antibodies employed in embodiments of the methods according to the invention are preferably monoclonal (mAb) αβ T cell receptor antibodies, and are generally commercially available or can be produced by known methods without undue experimentation. Non-monoclonal anti-αβ T cell receptor antibodies with suitable specificity and an efficacy similar to monoclonal αβ T cell receptor antibodies, or whose epitope overlaps that of the monoclonal antibody, are also suitable. It is also known that hybridomas producing monoclonal antibodies may be subject to genetic mutation or other changes while still retaining the ability to produce monoclonal antibody of the same desired specificity. The embodiments of the invention methods therefore encompass mutants, other derivatives and descendants of the hybridomas producing anti-αβ TCR mAbs. It is also known that a monoclonal antibody can be subjected to the techniques of recombinant DNA technology to produce other derivative antibodies, humanized or chimeric molecules or antibody fragments that retain the specificity of the original monoclonal antibody. Such techniques may involve combining DNA encoding the immunoglobulin variable region, or the complementarity determining regions (CDRs) of the monoclonal antibody with DNA coding the constant regions, or the constant regions plus framework regions, of a different immunoglobulin, for example, to convert a mouse-derived monoclonal antibody into one having largely human immunoglobulin characteristics. (See, for example, EP 184187A and GB 2188638A.) The embodiments of the invention also encompass humanized monoclonal antibodies to the αβ TCR epitopes. In a most preferred embodiment, fully human antibodies to human αβ TCR epitopes are employed in human recipients. These human antibodies can be polyclonal with suitable specificity and efficacy and, preferably, are human monoclonal antibodies.

As disclosed in the above applications incorporated by reference, a significant depletion of the T cell population was demonstrated at the end of immunodepleting regimens that included a combination of CsA and anti-αβ TCR antibodies, as well as repopulation of the recipient T cell repertoire once the treatment protocol had been withdrawn. None of the immunodepleted recipients showed signs of graft versus host disease (GVHD) or other signs of recipient immunodeficiency. It was also demonstrated that introduction of a 5-day, 7-day, or up to a 35-day short-term immunosuppressive protocol of combined CsA and anti-αβ TCR antibodies that were initially administered up to about 24 hours prior to transplantation in semi-allogeneic recipients of composite tissue allografts, resulted in induction and maintenance of donor-specific tolerance across a major histocompatibility barrier without the need for longer term, chronic immunosuppression. This was confirmed by the indefinite (>750 days) survival of the allografts in the recipients treated with the combined therapy. Using a protocol in which the combined CsA and anti-αβ TCR antibodies were initially administered at about the time of transplantation and daily for 5 days or 7 days only, tolerance was induced in fully-MHC mismatched allogaft transplants without the occurrence of GVHD, and donor specific tolerance was confirmed by the mixed lymphocyte reaction (MLR). The fully-allogeneic recipients have lived over 450 days after cessation of therapy, and continue to live.

Regardless of the immunosuppressive protocols employed, all of the recipients of the combined CsA/αβ-TCR mAb treatment were immunocompetent, as verified by a mixed lymphocyte reaction and the observation that the treated, allotransplanted recipients uniformly rejected third part grafts. Moreover, the immunocompetence of these recipients was verified biologically, as none of the animals over the entire range of protocols (including all recipients of semi-allogeneic and fully-allogeneic transplants and the combined treatment protocols) showed signs of disease, including viral infection or lymphoma formation, over 720 days post-transplant (which is a typical life span for a rat).

Mixed hematopoietic donor-recipient chimeric cells were identified in the peripheral blood of the semi-allogeneic and fully allogeneic allograft recipients by flow cytometry using our novel three-color immunostaining technique. In addition to CD4⁺ and CD8⁺ donor-recipient chimeric cells, CD90⁺ stem and progenitor cells and/or CD45RA⁺ B cell populations and/or CD90⁺/CD45RA⁺ B cell progenitor populations were found to be permissive cell populations facilitating tolerance induction (tolerance inducing cells, TICs). Without being bound by theory, it is believed that, since the vascularized bone marrow is an integral part of limb allografts, stem cells and progenitor cells of donor bone marrow origin become engrafted in the recipient lymphoid organs during immunosuppressive therapy according to embodiments of the invention, resulting in induction of hematopoietic mixed donor-recipient chimerism in the recipient. Circulating chimeric cells can be identified in the peripheral blood and/or lymphoid organs of the recipients by staining with a monoclonal antibody specific for a donor peripheral blood mononuclear cell (PBMC) antigen. A low level of hematopoietic chimerism as a mechanism of tolerance induction is supported by other studies in which we found that rats rejected uniformly skin flap allografts devoid of bone marrow despite the CsA/αβ-TCR mAb treatment. In contrast, skin grafts transplanted simultaneously with bone marrow of donor origin were accepted (over 80 days) across an MHC barrier.

In embodiments of the present invention, administration of the therapeutically effective amount of a combination of anti-αβ T cell receptor antibodies and the immunosuppressive agent capable of depleting T cells, preferably mature T cells, can be given prophylactically or therapeutically. By "prophylactic," it is meant the protection, in whole or in part, against allograft rejection. By "therapeutic," it is meant the amelioration of allograft rejection itself, and the protection, in whole or in part, against further allograft rejection. The antibodies and immunosuppressive drugs, as used herein, include all biochemical equivalents thereof (*i*.*e*., salts, precursors, the basic form, and the like).

In a preferred embodiment, the immunosuppressive agent and the anti-αβ T cell receptor antibodies are administered as a short course of therapy that can be initiated prior to transplantation, alternatively at transplantation, alternatively about one to about three days after transplantation and, preferably, continues for a short time period after transplantation.

In an embodiment of the invention that is particularly useful for semi-allogeneic transplantation, the immunosuppressive agent and the anti-αβ T cell receptor antibodies can be initially administered at about the time of transplantation to about 24 hours prior to transplantation, preferably about 12 hours to about 24 hours prior to transplantation. Administration of the immunosuppressive agent and the anti-αβ T cell receptor antibodies are then administered daily for about 100 days, about 50 days, about 35 days, about 21 days, about 14 days, preferably about 7 days or, especially, for about 5 days after transplantation.

In another embodiment of the invention that is particularly useful for fully-allogeneic transplantation, the immunosuppressive agent and the anti-αβ T cell receptor antibodies are initially administered during a period of time from about one hour prior to transplantation to at the time of transplantation. Administration of the immunosuppressive agent and the anti-αβ T cell receptor antibodies are then administered daily for about 100 days, about 50 days, about 35 days, about 21 days, about 14 days, preferably about 7 days or, especially, for about 5 days after transplantation. For fully allogeneic transplantation, it is more preferable initially to administer the immunosuppressive agent and the anti-αβ T cell receptor antibodies at about the time of transplantation, in order to avoid the occurrence of GVHD in these recipients.

In another embodiment, the immunosuppressive agent and the anti-αβ T cell receptor antibodies are first administered from one to three days after transplantation, and daily administration continues for a period of time of about 100 days, about 50 days, about 35 days, about 21 days, about 14 days, preferably for about 7 days or, especially, for about 5 days after transplantation.

In alternative embodiments, the immunosuppressive agent and the anti-αβ T cell receptor antibodies can be administered independently on a daily and/or non-daily basis during the treatment period of time, depending on the type of transplant, the type of donor, the condition of the recipient, and other factors, according to the judgement of the practitioner as a routine practice, without departing from the scope of the invention. In another embodiment, the immunosuppressive T cell deleting agent and the anti-αβ TCR⁺ T cell receptor antibodies are administered as a pharmaceutical composition that comprises both the agent and the antibodies.

The immunosuppressive agent(s) and/or the antibodies useful in embodiments of the invention can be a pharmaceutically acceptable analogue or prodrug thereof, or a pharmaceutically acceptable salt of the immunosuppressive agent(s) or antibodies disclosed herein, which are effective in inducing long-term, donor specific tolerance to allografts. By prodrug is meant one that can be converted to an active agent in or around the site to be treated.

Treatment will depend, in part, upon the particular therapeutic composition used, the amount of the therapeutic composition administered, the route of administration, and the cause and extent, if any, of the disease.

The antibodies and immunosuppressive agent(s) described herein, as well as their biological equivalents or pharmaceutically acceptable salts can be independently or in combination administered by any suitable route. The manner in which the agent is administered is dependent, in part, upon whether the treatment is prophylactic or therapeutic. Although more than one route can be used to administer a particular therapeutic composition, a particular route can provide a more immediate and more effective reaction than another route. Accordingly, the described routes of administration are merely exemplary and are in no way limiting. Suitable routes of administration can include, but are not limited to, oral, topical, subcutaneous and parenteral administration. Examples of parenteral administration include, but are not limited to, intravenous, intraarterial, intramuscular, intraperitoneal, and the like.

The dose of immunosuppressive agent, anti-αβ T cell receptor antibodies, and/or donor bone marrow cells administered to an animal, particularly a human, in accordance with embodiments of the invention, should be sufficient to effect the desired response in the animal over a reasonable time frame. It is known that the dosage of therapeutic agents depends upon a variety of factors, including the strength of the particular therapeutic composition employed, the age, species, condition or disease state, and the body weight of the animal. Moreover, the dose and dosage regimen will depend mainly on whether the compositions are being administered for therapeutic or prophylactic purposes, separately or as a mixture, the type of biological damage to the host, the type of host, the history of the host, and the type of immunosuppressive agents or biological active agent. The size of the dose will be determined by the route, timing and frequency of administration as well as the existence, nature and extent of any adverse side effects that might accompany the administration of a particular therapeutic composition and the desired physiological effect. It is also known that various conditions or disease states, in particular, chronic conditions or disease states, may require prolonged treatment involving multiple administrations. Therefore, the amount of the agent and/or antibodies must be effective to achieve an enhanced therapeutic index.

It is noted that humans are generally treated longer than mice and rats with a length proportional to the length of the disease process and drug effectiveness. The therapeutic purpose is achieved when the treated hosts exhibit improvement against disease or infection, including but not limited to improved survival rate of the graft and/or the host, more rapid recovery, or improvement in or elimination of symptoms. If multiple doses are employed, as preferred, the frequency of administration will depend, for example on the type of host and type of disease. The practitioner can ascertain upon routine experimentation which route of administration and frequency of administration are most effective in any particular case. Suitable doses and dosage regimens can be determined by conventionally known range-finding techniques. Generally, treatment is initiated with smaller dosages, which are less than the optimum dose of the compound. Thereafter, the dosage is increased by small increments until the optimum effect under the circumstances is reached.

The dose and dosage regimen will depend mainly on whether the compositions are being administered for therapeutic or prophylactic purposes, separately or as a mixture, the type of biological damage and host, the history of the host, and the type of immunosuppressive agent or biologically active agent. The amount must be effective to achieve an enhanced therapeutic index. It is noted that humans are generally treated longer than rats with a length proportional to the drug effectiveness. The doses may be single doses or multiple doses over a period of several days. Therapeutic purposes are achieved as defined herein when the treated hosts exhibit allograft tolerance, including but not limited to improved allograft survival rate, more rapid recovery, or improvement or elimination of transplantation-associated symptoms. If multiple doses are employed, as preferred, the frequency of administration will depend, for example, on the type of host and type of allograft, dosage amounts, and the like.

A further disclosure of immunosuppressive formulations suitable for use in the present invention is contained in the two patent applications incorporated by reference herein.

In our studies using a rat hindlimb (CTA) allograft transplantation model, a remarkably stable hematopoietic donor-recipient chimerism was observed in the recipients as evidenced by a peripheral blood chimeric cell level of about 20% to about 30% of circulating mononuclear cells, and greater than about 60% chimeric cells in lymphoid tissue. A study of the kinetics of the level of chimerism present in the peripheral blood of rat hindlimb CTA recipients receiving the combined immunosuppressive treatment and showing indefinite allograft tolerance was about 2% to about 3% of PBMC at about 7 days post transplant. The level of chimerism then rose to about 3% to about 6% at about 21 days post-transplantation, to about 10% at about day 35, and to about 15% to about 20% or more by about day 63 post-transplantation. At this stage, a stable multilineage (CD4, CD8 and CD45RA) chimerism was achieved.

Without being bound by theory, these finding suggested that the presence of certain tissue compartments in the composite tissue allografts may facilitate efficient engraftment of the donor hematopoietic cells and contribute to the observed long-term tolerance (over 750 days) across a major histocompatibility complex (MHC) barrier in a multitissue allograft. It is known that bone marrow stromal cells play a critical role in the formation of the hematopoietic microenvironment and support hematopoietic stem cell differentiation through the inter-cellular contact and secretion of various cytokines and growth factors. The hematopoietic microenvironment that is created by transplantation of marrow stromal cells, strains or crude bone marrow allows for the ectopic development of a hematopoietic tissue at the site of transplantation. Again, without being bound by theory, it is possible that microanatomic environment and stromal components in the bone component of the allograft can provide essential support elements that allow for the successful mismatched transplant without recipient preconditioning and contribute to the ability to induce a tolerant state allowing for stable chimerism.

We further discovered that enhancement of CTA survival can be achieved, with or without the development of enhanced mixed donor-recipient chimerism, by the combination of immunosuppressive agent and the anti-αβ T cell receptor antibodies and an additional implantation of a therapeutically effective amount of bone marrow cells from the same donor that provided the allograft. Moreover, we discovered that the enhanced allograft survival and mixed chimerism can be achieved by implantation of the donor bone marrow cells employing any delivery system including, but not limited to, an implantable, vascularizable bone from the allograft donor including the donor bone marrow; implantable crude donor bone marrow; an implantable donor bone marrow cell suspension; an implantable isolated subpopulation of nucleated donor bone marrow cells; and combinations thereof.

We discovered that the direct transplantation of the donor bone marrow in the crude form into the medullary cavity of the long bones of transplant recipient allows not only for the engraftment of the bone marrow cells, but also provides a natural matrix as an ideal microenvironment for cell engraftment for subsequent cell repopulation and trafficking. As described in the examples below, the engraftment of the donor specific bone marrow cells and their trafficking into the periphery was confirmed by flow cytometry, revealing 4.1 % and 7.4% of donor specific RT-1^{n-FITC}/CD4^{-PE} and RT-1^{n-FITC}/CD8^{-PE} chimeric cells, respectively, in the peripheral blood of recipients at day 63 after cessation of the immunosuppressive treatment protocol. The induction of this mixed, donor specific chimerism resulted a the significant extension of the skin allograft survival in recipients of the donor crude bone marrow. This method of crude bone marrow transplantation is simple, minimally invasive, does not require recipient preconditioning and can be easily implemented into clinical practice in humans.

We also discovered that implantation of a suspension of donor bone marrow nucleated cells implanted into the allograft recipient by direct intraosseous injection or by intravenous injection resulted in a strong stable mixed donor-recipient chimerism in the recipient.

We further discovered that isolated bone marrow nuclear cell subpopulations, such as pluripotent stem cells or progenitor cells, obtained by further processing the foregoing bone marrow suspensions, and implanted into allograft recipients, resulted in a strong, stable mixed donor-recipient chimerism in the recipient. In particular, intraosseous delivery of isolated subpopulations of stem and progenitor cells produced a high level of mixed chimerism (1.4% to 5.4%) maintained in the peripheral blood of the recipients for over 35 days post-transplantation.

Although in the rat hindlimb model, about 15% to about 20% chimeric cells were sufficient to achieve indefinite allograft tolerance, other protocols in other animals, including human recipients, may achieve a different peripheral blood level of chimeric cells that is sufficient to achieve indefinite allograft tolerance. Thus, an optimum level of chimerism for maintaining long-term allograft tolerance can vary and can be about 5% to about 50% of circulating PBMC, preferably about 10% to about 40%, more preferably about 15% to about 30%, most preferably about 20% to about 30% of circulating PBMC, without limitation, depending on the individual modality employed. The level of chimerism in lymphoid organs can be as high as about 60% or more and as low as about 25% or less.

In another embodiment of the invention, a method is provided for inducing donor-specific tolerance and/or mixed donor-recipient trimerism in an allograft transplant recipient, comprising the steps of: (a) administering to a recipient of an allograft a therapeutically effective amount of an immunosuppressive agent that depletes T cells; (b) administering to the recipient of the allograft a therapeutically effective amount of anti-αβ T cell receptor antibodies; (c) implanting a first allograft from a first allogeneic allograft donor into the recipient; (d) implanting a therapeutically effective amount of bone marrow cells from the first allograft donor into the recipient; (e) implanting a second allograft from a second allogeneic allograft donor into the recipient; and (f) implanting a therapeutically effective amount of bone marrow cells from the second allograft donor into the allograft recipient, wherein a state of trimerism is induced in the recipient. The first and second allogeneic allograft donors are preferably independently selected from semi-allogeneic donors; fully allogeneic donors; and combinations thereof, with respect to the recipient. Preferably, the state of trimerism in the recipient comprises a level of mixed donor and recipient cells of about 0.1 % to about 15%, preferably about 1% to about 10%, of circulating peripheral blood mononuclear cells.

This embodiment of the invention is particularly applicable to human transplantation. For example, a patient could receive a solid organ transplant, such as a heart, for example, from one unrelated donor and, later, could receive a kidney from a different unrelated donor. This scenario would produce a state of classical trimerism in the recipient, with co-existing recipient cells, and cells from the donor of the heart and from the donor of the kidney, all co-existing without organ rejection in the recipient. As a further permutation, the same patient could receive another kidney from yet another unrelated donor producing a state in which the cells of the recipient and of three independent donors co-exist in the recipient. This would be a state of "multichimerism."

In another non-limiting example, a patient could receive a solid organ transplant, such as a kidney, from a related donor that shares one or more MHC loci with the recipient, and a heart transplant from an unrelated donor. This scenario would produce a state of trimerism in the recipient, with cells expressing both donor and recipient antigens co-existing with cells from the related donor. A later transplant from a different related donor that shares a different MHC locus with the recipient, or unrelated donor would produce a state of multichimerism.

In another embodiment of the invention, a method is provided for maintaining a level of mixed donor-recipient chimerism in an allograft transplant recipient, with the goal of prolonging allograft tolerance. By the method, an optimum level of chimerism for maintaining long-term allograft tolerance can be determined by measuring an optimal level of chimeric cells in the recipient not undergoing rejection of the allograft. When an optimal level is achieved, the chimeric cells can be harvested from the recipient and stored or, optionally, propagated in cell culture prior to storage. If the recipient later shows signs of allograft rejection, or if the level of chimeric cells is decreasing, or reaches or falls below a minimum level considered sufficient to maintain allograft tolerance, the harvested stored cells are then available to reconstitute the recipient chimeric cells to maintain the tolerant state.

Optionally, the method can include readministering an effective amount of the immunosuppressive agent and/or the anti-αβ T cell receptor antibodies in addition to the chimeric cells. The harvested stored chimeric cells can be administered to the recipient by any method described above including, but not limited to, direct intraosseous injection into a recipient bone marrow cavity; direct intraosseous injection into a bone marrow cavity of an implanted donor bone allograft; intravenous injection into the recipient; and the like, and combinations of the foregoing.

While individual recipients will vary in the optimal and minimum levels of chimeric cells, the minimum level of mixed donor and recipient cells has been found in the rat model to be about 5% to about 20% of circulating peripheral blood mononuclear cells, and the optimal level has been found to be about 20% to about 50% of circulating peripheral blood mononuclear cells. Individual human recipient equivalents can be established without undue experimentation, and are envisioned to be about the same as in the rat model here described.

The harvested chimeric cells can be obtained from any recipient tissue or lymphatic organ, but are preferably obtained from the peripheral blood. For example, chimeric cells can be obtained by blood donation from allograft recipients on a regular basis such as, but not limited to, monthly, bimonthly, semi-annually, annually, and the like, for any period of time during which optimal levels are maintained.

Peripheral blood mononuclear cells can be separated from the peripheral blood by methods that are well known in the art, and employed for later reconstitution of the recipient. Alternatively, the chimeric cell subpopulations can be separated from the peripheral blood/peripheral blood mononuclear cells by any suitable cell separation method, such as those described above, or the like. Alternatively, the whole peripheral blood can be frozen and stored for later infusion into the recipient.

The harvested PBMC cells and/or chimeric cell subpopulations can be directly stored at - 196°C in liquid nitrogen, or by similar known means. Preferably, the cells are stored in a medium suitable for cryogenic preservation. Alternatively, the harvested cells can undergo expansion in culture prior to storage, using appropriate culture media, feeder cell layers, and the like, that will allow proliferation of the cells. For example, a suitable method for culturing harvested rat cells employs a MyeloCult medium (StemCell Technologies, Vancouver, BC) and rat cell feeder layers in tissue culture dishes. The harvested chimeric cells can be divided and a portion of the harvested cells used to reconstitute the recipient, with the remaining portion stored for later use.

Therefore, according to embodiments of the present invention, mixed donor-recipient chimeric cells are harvested from the allograft recipient. Preferably, the recipient is human.

In one embodiment, the invention provides a method for obtaining mixed donor-recipient chimeric cells, comprising the steps of (a) administering to an allograft recipient a short-term immunosuppressive regimen that depletes about 50% to about 99.9% of recipient T cells circulating in the peripheral blood of the recipient; (b) implanting an allograft from a donor into the recipient; (c) allowing the development of mixed donor-recipient chimeric cells in the recipient after completion of the immunosuppressive regimen; and (d) harvesting the chimeric cells from the recipient. Preferably, the chimeric cells are stored after harvesting, by cryogenic or other known cell storage methods. More preferably, the harvested chimeric cells are propagated in culture to expand their numbers prior to storage.

In a preferred embodiment of the invention, after harvesting, the expression of surface histocompatability antigens of the chimeric cells is determined and the chimeric cells are accordingly categorized as to histocompatibility antigen expression "type". These categorized chimeric cells can then be stored to become part of a library of similarly typed stored chimeric cells from multiple allograft transplant recipients, providing a multiplicity of histocompatability types. Preferably, the chimeric cells can be obtained from transplant recipients on a world-wide basis, ultimately providing a library or universal chimera bank containing every possible combination of histocompatability types.

The categorized chimeric cells obtained as described above can be used to induce and/or maintain immunological tolerance in a naive recipient requiring an allogeneic trransplant, for example, or in a transplant recipient requiring an additional transplant. According to this embodiment of the invention, a method is provided for inducing allograft tolerance and/or mixed donor-recipient chimerism in an allograft recipient, comprising the steps of (a) determining a histocompatibility antigen type of an allograft recipient; (b) determining a histocompatibility antigen type of an allograft donor; (c) selecting chimeric cells from a library of chimeric cells, wherein the selected chimeric cells represent a best match for the histocompatibility antigen types of both the recipient and the donor; (d) administering to an allograft recipient a short-term immunosuppressive regimen that depletes about 50% to about 99.9% of recipient T cells circulating in the peripheral blood of the recipient; (e) administering to the recipient a therapeutically effective amount of the selected chimeric cells; (f) implanting an allograft from a donor into the recipient, wherein a combination of steps (d) and (e) results in recipient immunological tolerance to the allograft.

Optionally, the method further comprises the individual optional steps of monitoring a level of circulating chimeric cells in the allograft recipient or monitoring the allograft for a visual or histological sign of rejection; readministering a therapeutically effective amount of the selected chimeric cells to the recipient if the level of circulating chimeric cells declines to a preselected minimum level or a sign of rejection is observed; and/or readministering a short-term immunosuppressive regimen if the level of circulating chimeric cells declines to a preselected minimum level or a sign of rejection is observed. In a preferred embodiment, the preselected minimum level of circulating chimeric cells is a minimum level for preventing onset of allograft rejection.

In an alternative embodiment, the selected chimeric cells can comprise chimeric cells produced as the result of fusion of cells of different histocompatibility antigen types, to produce cells having surface histocompatibility antigens of two or more of the cell types. Such manipulation can take place *ex vivo* by known cell fusion methods such as, but not limited to, electrofusion techniques or polyethylene glycol (PEG) fusion, and the like.

In another embodiment of the invention, a library of chimeric cells is obtained. Preferably chimeric cells are harvested from an allograft recipient or produced by cell fusion methods. The method for compiling a library of chimeric cells includes determining expression of surface histocompatability antigens of the chimeric cells; optionally culturing the harvested chimeric cells to obtain an expanded population of the chimeric cells; and storing the chimeric cells.

The invention also provides a library of chimeric cells and an animal model of chimeric recipients produced according to the embodiments described above. The invention is particularly useful for allograft transplantation in human recipients, and also encompasses rodent and/or higher mammal recipients, and the like.

### EXAMPLES

To illustrate embodiments of the method of the invention for inducing allograft tolerance and/or mixed donor-recipient chimerism in an allograft recipient, the following examples employ several allograft transplantation models, immunosuppressive protocols, and delivery systems for donor bone marrow and/or bone marrow cells, including isolated donor stem and progenitor cell populations

The examples described herein are not intended to be limiting, as one skilled in the art would recognize from the teachings hereinabove and the following examples that, for example, other immunosuppressive regimens, including other immunosuppressive agents, other anti-αβ T cell receptor antibodies, other dosage and treatment schedules, other methods of bone marrow delivery, other isolation methods for donor stem and progenitor cell recovery, other sources of donor stem and progenitor cells, other animal and/or humans, and the like, all without limitation, can be employed, without departing from the scope of the invention as claimed.

### Example 1

### Extended Survival of Allogeneic Skin Transplants in Recipients Under a Combined CsA/αβTCR mAb Immunosuppressive Treatment Protocol and Direct Transplantation of Crude Donor Bone Marrow Into Recipient Bone Marrow Cavity

In this example, 43 skin graft transplantations were performed in 9 animal groupings, described below, between isogeneic [Lewis to Lewis (LEW, RT-1¹)] and semi-allogeneic [Lewis x Brown Norway (LBN →F1, RT-1¹⁺ⁿ) to Lewis] rat strains under anti-αβ-TCR mAb and CsA treatment. The allogeneic skin graft recipient also received a crude bone marrow transplantation from the same donor into a recipient bone marrow cavity. The use of combined protocol of CsA/αβ-TCR mAb and the crude bone marrow transplantation resulted in the extension of skin allograft survival up to 65 days after cessation of the immunosuppressive treatment (p<0.05).

The following animals, reagents, assays and techniques were employed in Example I.

### I. Animals

Inbred male rats weighting 150-175 grams were purchased from Harlan Sprague-Dawley, Indianapolis, IN). Lewis rats (LEW) served as the recipients of skin and crude bone marrow allografts from Lewis-Brown Norway donors (LBN). The animals were caged at room temperature on a 12-hour light/dark cycle with free access to food and water. All animals received humane care in compliance with the Guide for the Care and Use of Laboratory Animals published by the National Institute of Health in the facility accredited by the American Association for the Accreditation of Laboratory Animal Care.

### II. Transplantation Techniques

Skin grafting and crude bone marrow transplantation were performed at the same session from the same donor. Intraperitoneal pentobarbital (50 mg/kg) was used as an analgesic during the transplantation procedure.

### A. Skin Grafting Transplantation Technique

Skin grafting was performed according to the technique described by Billingham (Billingham R E. and P. B. Medawar. J. Ex. Biol. 1951; 28: 385-402). Briefly, full thickness skin grafts 16 mm in diameter were taken from the donors. Graft beds were prepared by excising 18 mm circles on the lateral dorsal thoracic walls of the recipients. Care was taken to remove *perniculous carnosum* from the grafted skin. Both sides of the thoracic wall were used for allogeneic grafts and the mid-sternum was used for syngeneic grafts. All grafts were separated by a 10 mm skin bridge. A standard compressive dressing and adhesive bandage was used for 7 days.

### B. Crude Bone Marrow Transplantation Technique

Donor-origin bone marrow was transplanted in its crude form containing all natural microanatomical components of the bone marrow including pluripotent stem cells and progenitor cells and extracellular matrix.
(a). Harvesting of the crude bone marrow from the donor: The metaphyseal region of the right tibia was approached from an anterior incision. Next, on the anteromedial cortex of the tibia, a 3 mm window was created using a 1/32-inch drill. Following decortication, the contents of the metaphyseal part of the bone were removed with a bone curette and placed in a container cooled in ice. Next, the bone rongeur was used to harvest intramedullary content along the diaphysis. The weight of the harvested BM was measured before transplantation.
(b). Transplantation of the crude BM to the recipient: The recipient's tibia was opened in a similar fashion as the donor's bone, exposing the anteromedial surface of the right tibia, followed by creation of the cortical window. The recipient's cancellous bone and content of the BM cavity were totally removed to create an appropriate "space" for the BM engraftment. The harvested bone marrow of donor origin was next packed into the "empty" medullary cavity of the recipient's tibia and sealed with bone wax.

### III. Immunosuppressive Treatment Protocols

Treatment protocols included monotherapy with CsA alone or anti-αβ TCR monoclonal antibody (anti-αβ-TCR mAb) alone, or a combination of CsA and anti-αβ TCR monoclonal antibody (CsA/αβ-TCR mAb). Both CsA and anti-αβ-TCR mAb were administered 12 hours before transplantation and continued up to 7 days or 35 days in the combined therapy group.

Cyclosporine A (Sandoz Pharmaceutics Inc., East Hanover, NJ) was dissolved daily in PBS (Fisher Scientific, Pittsburgh, PA) to a concentration of 5 mg/ml and administered subcutaneously (s.c.) to recipient animals. Animals under CsA treatment received a dose of 16 mg/kg/day (s.c.) administered 12 hours before transplantation and daily thereafter for the first week, 8 mg/kg/day during the second week, 4 mg/kd/day for the third and fourth week, and 2 mg/kg/day for the fifth week. Intraperitoneal (i.p.) injection of anti-αβ TCR mAb (clone R73, Pharmingen, San Diego, CA) (250 µg) was administered 12 hours before transplantation, and daily thereafter for the first week. The dosage of anti-αβ TCR mAb was then tapered to 50 µg at the end of the first week and was given every 2 days during the second week and every 3 days during the last 3 weeks.

The 7-day protocol was similar. Animals under CsA treatment received a dose of 16 mg/kg/day (s.c.) administered one hour or 12 hours before transplantation (when semi-allogeneic transplants were performed) and daily thereafter for 7 days. Intraperitoneal injection of anti-αβ TCR mAb (250 µg) was administered one hour or 12 hours before transplantation (when semi-allogeneic transplants were performed), and daily thereafter for 7 days.

In each of the foregoing protocols, when fully allogeneic transplants were performed, both the CsA and anti-αβ TCR mAb treatments were initially administered at the time of transplantation, at the time of clamp release.

### IV. Treatment Groups

The treatment groups, treatment protocols, and graft survival are illustrated in **Table 1.** Multiple trials were performed according to the protocols. The immunosuppressive treatment was administered using the 35 protocol or the 7 day protocol. The amount of crude donor bone marrow transplanted ranged from 20 mg to 100 mg.

The following treatment groups were employed in one series of transplants between semi-allogeneic donors/recipients:
Group 1: Skin Isograft Control (n=6): Skin grafts were transplanted between LEW rats without immunosuppressive treatment before or after transplantation.
Group 2: Skin Allograft Control (n=6): Skin allografts were transplanted from LBN donors to LEW recipients without immunosuppressive treatment before or after transplantation.
Group 3. Skin and Bone Marrow Allograft Control (n=6): Both the skin and the crude bone marrow were transplanted from donor LBN to recipient LEW rats without immunosuppressive treatment before or after transplantation.
Group 4. Skin Allografts + CsA (n=3): Skin allografts were transplanted from LBN donor to LEW recipients. Animals in this group received 5 weeks of CsA treatment following skin transplantation.
Group 5. Skin Allografts + anti-αβ TCR mAb (n=3): Skin allografts from LBN donors were transplanted to LEW recipients. Animals received 5 weeks of anti-αβ TCR mAb treatment following skin transplantation.
Group 6. Skin Allograft + CsA + anti-αβ TCR mAb (n=5): Skin allografts from LBN donors were transplanted to LEW recipients. Animals received 5 weeks of combined CsA and anti-αβ TCR mAb treatment following skin transplantation.
Group 7. Skin Allograft + BM + CsA (n=5): Skin and the crude bone marrow from the same LBN donors were transplanted to LEW recipients. The recipient received 5 weeks of CsA treatment following transplantation.
Group 8. Skin Allograft + BM + anti-αβ TCR mAb (n=4): Skin and the crude bone marrow from the same LBN donors were transplanted to LEW recipients. The recipient received 5 weeks of anti-αβ TCR mAb treatment following transplantation.
Group 9. Skin Allograft + BM + CsA + anti-αβ TCR mAb (n=5). Combined CsA and anti-αβ TCR mAb protocol was applied for 5 weeks following the skin and the crude bone marrow allograft transplantation from the same LBN donors to LEW recipients.

### V. Clinical Assessment of Allograft Rejection

The physical signs of skin allograft rejection, such as erythema, edema, loss of hair, scaling of the skin, and desquamation were evaluated on a daily basis. Rejection was defined as the destruction of over 80 percent of the graft.

### VI. Assessment of Graft Versus Host Disease (GVHD)

All animals were evaluated for the appearance of GVHD. The clinical criteria, such as diffuse erythema (particularly of the ear), hyperkeratosis of the foot pads, dermatitis, weight loss, generalized unkempt appearance, or diarrhea were monitored daily. An animal was considered to exhibit acute GVHD if at least four of the above signs were observed. The diagnosis of GVHD was confirmed by routine hematoxylin and eosin histologic staining performed on formalin-fixed skin, tongue, liver and small intestine samples collected at the time of occurrence of the first signs of GVHD. Grading of GVHD was performed in blinded fashion according to previously described histologic criteria (Sale, G.E. et al. Am. J. Surg. Pathol. 1979; 3: 291; Saurat, J.H. et al. Br. J. Dermatol. 1975; 93: 675) and were assessed by a histopathologist.

### VII. Flow Cytometry Analysis

Flow cytometry (FC) analysis was performed according to the manufacturer's protocol (Becton Dickinson, San Diego, CA) with minor modifications. The blood samples of transplant recipients were collected into heparinized tubes on the following days post-transplantation: 0, 7, 21, 35, 63 and at the time of initial signs of clinical rejection. The peripheral blood mononuclear cells (PMBC) were incubated for 20-30 minutes in the dark at room temperature with 5 µL of a mixture of mouse anti-rat monoclonal antibodies conjugated with fluorescein isothiocyanate (FITC) or phycoerythrin (PE) against CD4^{-FITC} (Clone OX35), CD8a^{-PE} (Clone OX8), αβ TCR^{-FITC} (Clone R73), CD45RA^{-PE} (Clone OX-33). After incubation, samples were resuspended in FACS Lysing solution (Becton Dickinson), incubated for 20 minutes in the dark at room temperature, centrifuged at 1500 rpm for 5 minutes, washed twice with washing buffer (phosphate-buffered saline, PBS, without Mg⁺⁺, Ca⁺⁺, 0.1% bovine serum albumin, 0.05% NaN₃), fixed with 2% paraformaldehyde solution, covered with aluminum foil and stored at 4°C until flow cytometry assessment.

### VIII. Donor-Specific Chimerism Evaluation by FC

For the determination of donor-specific lymphoid chimerism in the peripheral blood of recipients, combinations of mouse anti-rat CD4^{-PE} or CD8a^{-PE} with RT-1ⁿ (Brown Norway MHC class I, Clone MCA-156, Serotec, Kidlington, UK) were applied. For the donor-derived, RT-1ⁿ-positive staining cells, purified anti-rat CD-32 (FcγII Block Receptor) antibody (1:20) was added first to block the Fc-mediated adherence of antibodies. After 3-4 minutes pre-incubation, samples were further incubated with 5 µL of mouse anti-rat RT-1ⁿ for 30 minutes at 4°C Then samples were washed twice in washing buffer and stained with goat anti-mouse FITC conjugated IgG (rat adsorbed; Serotec). This was followed by the incubation with CD4^{-PE} or CD8^{-PE} conjugated mouse anti-rat monoclonal antibody. After incubation, samples were processed as described above. The negative control included isotype-matched antibodies and/or PBS-incubated samples. FC analyses were performed on 1 x 10⁴ mononuclear cells by using FACS *Scan* (Becton Dickinson) and CellQuest software.

### IX. Statistical Evaluation

Treatment groups were compared on survival using the log-rank test, and survival times were estimated using the Kaplan-Meier method. The level of chimerism, and efficacy of the immunosuppressive treatment were compared by the independent samples t test. Differences were considered statistically significant at p<0.05.

**The Survival of the Skin Allografts.** The survival of skin allografts in each transplant group is presented in Table 1. The combined protocol of CsA and anti-αβ TCR mAb applied to the recipients of skin allografts without bone marrow significantly extended allograft survival when compared to the allograft controls without treatment (p<0.05). However, following cessation of Cs/αβ TCR mAb therapy, all allografts were rejected within 20 days (p<0.05). It was demonstrated that monotherapies, combined with crude donor bone marrow transplantation, resulted in extended survival up to 21 days (under CsA) and up to 10 days (under anti-αβ-TCR mAb). Monotherapy with CsA alone extended skin transplant survival over 7 days, whereas monotherapy with anti-αβ TCR mAb alone resulted in rejection at the same time as the rejection controls without treatment. When skin allograft transplantation and the crude bone marrow transplantations were performed in one procedure, the skin allograft transplant survival was extended by over 60 days (p<0.05) after cessation of the CsA/αβ TCR mAb protocol.

Extended skin allograft survival was achieved in animals receiving crude bone marrow under short term (7 days only) and longer term (5 weeks) CsA/αβ-TCR mAb treatment protocol.

**Determination of GVHD**. None of the allogeneic transplant recipients showed clinical signs of GVHD.

**Flow Cytometry Analysis of *In Vivo* Depletion of αβ TCR⁺ Cells.** As illustrated in **Figure 1****,** FC determination of αβ TCR expression on lymphocytes harvested from sentinel (untransplanted animals treated with CsA/αβ TCR mAb) and transplanted animals treated with CsA/αβ TCR mAb showed >90% depletion of the αβ TCR⁺ cell population 7 days after immunosuppressive treatment cessation (day 42 post-transplantation). Repopulation of αβ TCR⁺ cell populations to the pre-transplantation level was observed 28 days after cessation of the immunosuppressive treatment (day 63 post-transplantation). The population of CD4⁺ and CD8⁺ cells in the peripheral blood of the sentinel and transplanted recipients was significantly reduced at day 7 (75% of CD4⁺ and 55% of CD8⁺) and a gradual repopulation of both cell subpopulations was seen at day 63 (data not shown).

**Mixed Donor-Recipient Chimerism in Recipients of Crude Donor Bone Marrow.** Two-color flow cytometry analysis of the donor-specific macro-chimerism was performed on PBMC of recipients of crude donor bone marrow and skin allografts. The dot-plot results of the lymphoid cell subpopulations obtained from quadrangle analytical gates at day 65 after allotransplantation demonstrated the presence of the multilineage donor-specific chimerism ranging from 18-29% (RT-1ⁿ positive cells). Examination of two-color stained RT-1^{n-FITC}/CD4^{-PE} and RT-1^{n-FITC}/CD8^{-PE} peripheral lymphocytes revealed 4.1% and 7.4%, respectively, of double positive CD4 and CD98 T cell subpopulations, illustrated in **Figure 2****, A** and **B,** respectively. The expression of RT-1ⁿ antigen on non-CD4 and non-CD8 positive T cell populations suggests the existence of donor/recipient chimeric residents not evaluated with the PE-conjugated mAb against surface specific antigen (B-lymphocytes and monocytes). Therefore, the donor crude bone marrow transplant inoculated directly into the recipient's bone marrow cavity allowed for optimal engraftment, repopulation and subsequent trafficking outside the bone marrow cavity into the periphery, resulting in donor specific chimerism.

In trials employing different amounts of crude donor bone marrow ranging from 20 mg to 100 mg, it was found that chimerism and extended allograft survival was achieved when the weight of transplanted crude bone marrow was greater than about 50 mg.

**TABLE 1**

| **Transplant Groups** | **Transplantation Procedure¹** | **Immunosuppressive Treatment Protocol** | **Days of Survival After Cessation of Treatment** | **Mean ± SD** |
|---|---|---|---|---|
| Group 1 (n=6) Isograft | Skin | None | Indefinite | Indefinite |
| Group 2 (n=6) Allograft | Skin | None | 6, 8, 7, 6, 7, 8 | 7 ± 0.9 |
| Group 3 (n=6) Allograft | Skin | None | 7, 8, 9, 6, 7, 8 | 7.5 ± 1 |
| | | | | |
| Group 4 (n=3) Allograft | Skin | CsA | 15, 16, 15 | 15 ± 0.5 |
| Group 5 (n=3) Allograft | Skin | αβ TCR mAb | 7, 9, 10 | 8.6 ± 1.5 |
| Group 6 (n=5) Allograft | Skin | CsA + αβ TCR mAb | 20, 21, 20, 19, 22 | 20.4 ± 1.1 |
| | | | | |
| Group 7 (n=5) Allograft | Skin + Crude BM | CsA | 20,22,24,21,20 | 21.4 ± 1.7 |
| Group 8 (n=4) Allograft | Skin + Crude BM | αβ TCR mAb | 9, 12, 10, 11 | 10.5 ± 1.3 |
| Group 9 (n=5) Allograft | Skin + Crude BM | CsA + αβ TCR mAb | 60, 65, 60, 67, 71 | 65 ± 4.7² |

| | | | | |
|---|---|---|---|---|
| ¹ All tranplantations except in group 1 were performed with LBN donors and LEW recipients. ² p<0.05 | | | | |

### Example 2

### Enhancement of CTA Survival by Intraosseous Delivery of Donor-Derived Stem Cells and Progenitor Cells

This example illustrates that increases in the level of hematopoietic chimerism can improve the survival and maintenance of CTA transplants without immunosuppressive therapy. The exemplary design included transplantation of the rat hindlimb allograft (LBN to LEW) concomitant with the direct intraosseous transplantation of bone marrow stem and progenitor cells isolated from the same donor. The following techniques and treatment regimens were employed.

### I. Hindlimb Transplantation Technique

Transplantations of hindlimbs between donor and recipient were performed under pentobarbital (50 mg/kg intraperitoneal) anesthesia using a standard microsurgery procedure (Press, B.H.J. et al. 1986. Ann. Plast. Surg. 16: 313-321). Briefly, a circumferential skin incision was made in the proximal one third of the right hindlimb. The femoral artery and vein were dissected, clamped, and cut proximal to the superficial epigastric artery. The femoral nerve was dissected and cut 1 cm distal to the inguinal ligament. The biceps femoris muscle was transected to expose the sciatic nerve. The nerve was then cut proximal to its bifurcation.

The donor was prepared in a similar way. The right hindlimb was amputated at the midfemoral level. The donor limb was attached to the recipient limb by a 20-gauge intramedullary pin and a simple cerclage wire. All large muscle groups were sutured in juxtaposition. The iliac vessels of the donor and femoral vessels of the recipient were anastomosed under an operating microscope with 10-0 sutures by using a standard end-to-end microsurgical anastomosis technique. The femoral and sciatic nerves were repaired by using a conventional epineural technique with four 10-0 sutures.

### II. Purification of CD90⁺ Stem and Progenitor Bone Marrow Cells

Bone marrow cells were isolated from donors using flushing methods. Briefly, freshly isolated femur and tibia were washed with sterile, cold PBS (without Mg⁺⁺ and Ca⁺⁺) supplemented with 1.0% bovine serum albumin (BSA). Two contralateral ends of the bones were cut and residual bone marrow cells were flushed out from the bone marrow cavity using a syringe-based pump system. After lysis with NH₄Cl/TRIS sterile hemolytic buffer for 5 minutes, nucleated marrow cells were then washed (PBS, 1.0 BSA) twice and counted to obtain a final concentration of 1 x 10⁶ nucleated cells/ml.

For isolation of the CD90⁺ cells, isolated nucleated bone marrow cells were incubated with FITC conjugated mouse anti-rat CD90 mAb (OX-7, Pharmingen) for 30 minutes in the dark at 4°C. After incubation, samples were washed twice with washing buffer, incubated for 30 minutes in the dark at 4°C with magnetic beads-conjugated mouse anti-FITC mAb, washed, and placed into MiniMACS separation columns (Miltenyi Biotec, Auburn, CA). The CD90⁺ cells were collected and their viability and number assessed by counting of the cells incubated with trypan blue. The assessment of the efficacy of purification of the MACS positively selected cells was accomplished by FC evaluation of the level of double positive CD90^{FITC} (Clone OX7)/RT-1ⁿ cells.

**Figure 3** illustrates FC analysis of the CD90⁺ antigen expression on the surface of stem and progenitor cells from donor bone marrow before and after selection using a magnetic MiniMACS separating system. Figure 3A shows CD90⁺ cells as the small peak, and the remainder of the bone marrow nucleated cells as the larger peak, prior to selective separation of the CD90⁺ cells from a suspension of bone marrow cells. The CD90⁺ cells comprised about 22% of the bone marrow nucleated cells. Figure 3B shows the purity of separation of the CD90⁺ cells (large peak). Less than 5% were CD90⁻. Over 95% of analyzed cells expressed the CD90 antigen, indicating high efficacy of the selection.

### III. Intraosseous Injection Of Donor Derived Stem And Progenitor Cells

A 50 µL high-purity suspension containing 8-12 x 10⁵ CD90⁺ stem and progenitor cells was injected directly into the bone marrow cavity of the recipient's contralateral tibia just before transplantation of the opposite hindlimb. No immunosuppressive protocol was given. **Figure 4A** illustrates injection of the stem and progenitor cells into the bone marrow cavity of the recipient's left tibia. Following injection, the right hindlimb from the same donor was transplanted to the recipient. (**Figure 4B**)

**Survival of Limb Allografts Following Preooperative Intraosseous Injection of Donor Stem and Progenitor Cells.** As shown in the limb allograft survival chart illustrated in **Figure 5****,** recipients receiving the allograft and also receiving intraosseous injection of donor CD90⁺ stem and progenitor cells, had a significant biological extension (up to 15 days) of limb allograft survival without immunosuppressive therapy (p<0.05), compared to recipients receiving the allograft only. This survival extension correlates with a transient chimerism level of 3.4% of CD4⁺ T cells of donor origin in the peripheral blood of the recipients, illustrated by the FC analysis of **Figure 6****.** A two-color FC analysis at the 14^{th} day after limb allograft transplantation revealed transient chimerism in the allograft rejection control group without treatment (0.6%, **Figure 6A**) and a higher level (3.4%, **Figure 6B**) of double positive RT-1¹⁺ⁿ/CD4⁺ chimeric cells in the peripheral blood of the limb recipients treated with the intraosseous injection of the CD90⁺ stem and progenitor cells at the time of transplantation.

### Example 3

This example illustrates a comparison of the level of donor/recipient chimerism in the hindlimb transplantation model, after intraosseus transplantation of donor stem and progenitor cells or intravenous injection of the same number of donor stem and progenitor cells.

**Intraosseous Transplantation of Donor Stem and Progenitor Cells Produces Long Term Donor-Specific Chimerism.** In a further example, 50 µL of a high purity (>95%) suspension containing 35-40 x 10⁶ donor stem and progenitor cells were obtained as described above. The same number (35-40 x 10⁶) of cells was injected intravenously into the epigastric vein in one group of recipient rats and directly into the tibial bone marrow cavity in the another group of recipient rats. Hindlimb transplants were then performed as described above. At day 35 post-transplant, flow cytometry revealed high levels of multilineage donor-specific lymphoid chimerism (**Figure 7****, B1-B3**) in the peripheral blood of the recipients receiving direct intraosseous donor stem and progenitor cells at the time of transplantation, which was maintained over 35 days (still under evaluation). In contrast, intravenous injection of the donor stem and progenitor cells at the time of transplantation resulted in low-level, transient (up to 5 days) chimerism (**Figure 7****, A1-A3**). These results confirm the importance of the microanatomic environment and stromal compartment in the efficacy of stem and progenitor cell engraftment. The results show that direct delivery of the stem and progenitor cells into the bone marrow cavity results in increased efficacy of donor cell engraftment and augmentation of mixed chimerism.

### Example 4

### Chimerism in Vascularized Skin/Vascularized Bone Transplantation

A vascularized embodiment of CTA comprising vascularized skin with subcutaneous fat (VS), and vascularized bone (VB) with cartilage and bone marrow was employed. The allograft transplantations were carried out across MHC semi-mismatched donors and recipients (LBN; RT-1¹⁺ⁿ → LEW; PT-1¹) and MHC fully mismatched donors and recipients (BN; RT-1ⁿ → LEW; RT-1¹), as illustrated below. Ten animals were employed in each group. The treatment protocols and surgical procedures are also described below.

| **Type of Graft** | **Donor** | **Recipient** |
|---|---|---|
| Isogeneic graft | Lewis (LEW; RT-1¹) | Lewis (LEW; RT-1¹ |
| Semi-allogeneic graft | Lewis Brown Norway (LBN; RT-1¹⁺ⁿ) | Lewis (LEW; RT-1¹) |
| Fully allogeneic graft | Brown Norway(BN; RT-1ⁿ) | Lewis (LEW; RT-1¹) |

### I. Immunosuppressive Treatment Protocol

All recipients of different combinations of the vascularized skin and (vascularized) bone allograft (VSBA) transplants were given the immunosuppressive therapy, whereas isograft controls received no treatment. A 7-day immunosuppressive treatment protocol, similar to that described in Example 1, was employed. Briefly, the treated animal groups received 16 mg/kd/day s.c. of CsA and 250 µg/day i.p. of anti-αβ TCR mAb daily for 7 days. The first treatments were given one hour prior to transplantation. Isogeneic transplant recipients received no immunosuppressive therapy.

### II. Surgical Procedures

### A. Vascularized Skin Grafting Procedure

Vascularized skin allograft transplantation was performed according to the technique described by Strauch *et al*. (Strauch, B. and D.E. Murray. Plast. Recons. Surg. 1967; 40: 325-329). Briefly, a standard 4 x 6 cm template was used to mark the flap borders both in the donor and the recipient. The donor skin flap was elevated on the superficial epigastric branch of the femoral artery and vein of the donor, and end-to-end anastomoses were performed between the donor's and recipient's femoral arteries and veins using standard microsurgical techniques.

### B. Vascularized Bone Transplantation

A vascularized femoral bone allograft was harvested on the femoral artery and vein of the donor, preserving supplying collateral vessels. The bone allograft was transferred to the recipient's groin region and end-to-end anastomoses between donor's and recipients femoral arteries and veins were performed using standard microsurgical techniques.

**Figure 8A** is a schematic representation of the vascularized skin and bone allograft (VSBA).combining superficial epigastric skin flap and vascularized femoral bone allograft.

**VSBA Transplants are Accepted Across Semi-Allogeneic and Fully-Allogeneic MHC Barriers.** The VSBA isografts, and semi-allogeneic and fully allogeneic grafts in recipients receiving the combined immunosuppressive therapy, showed indefinite (over 200 days) survival of both the skin and bone components of this tissue assembly, whereas VSBA allograft controls without immunosuppressive therapy rejected uniformly within 7 days (not shown). For example, **Figure 8B** illustrates a Giemsa stained (donor) vascularized bone marrow isograft 7 days after transplantation into the recipient, showing over 99% viability of the bone marrow cells in the bone transplants. **Figure 8C** shows complete acceptance of a vascularized skin allograft in a representative fully allogeneic VSBA recipient transplanted across a major MHC barrier (BN→LEW) at day 63 after cessation of immunosuppression. Biopsy of the skin (hematoxylin and eosin stained) shows preserved dermis and epidermis and no histological signs of rejection (**Figure 8D**).

**Trafficking of Bone Marrow-Derived Cells From VSBA Transplant Recipients to Donor Bone Marrow.** **Figures 8E** and **8F** show immunohistostaining of frozen sections of the bone marrow tissue, and FC analysis of the bone marrow cells, respectively, taken from the donor vascularized bone transplant in the VSBA of a representative fully allogeneic recipient at day 63 after cessation of immunosuppression. The analysis showed replacement of the CD90⁺ stem cells of the donor by the recipient's CD90⁺ cells. More than 50% of the cells were CD90⁺ and RT-1^{L} (related to LEW MHC class I) positive cells of the recipient origin. These results proved the trafficking of the donor bone marrow derived stem cells between the recipient and donor bone and confirmed the viability of the transplanted vascularized bone marrow. Similar results were obtained in semi-allogeneic transplants (data not shown).

### Example 5

### Development of a Trimeric Recipient From Fully Allogeneic Transplants From Genetically Unrelated Donors

The VSBA transplantation surgical procedure was performed as described in Example 4. Each Lewis (LEW, RT-1¹) rat received a vascularized bone allograft, including a vascularized skin flap, from two genetically unrelated allograft donors, i.e., Brown Norway (BN, RT-1ⁿ) and ACI (A x C Irish, RT-1^{a}) rats. Both VSBA transplantations were performed during the same operative procedure.

Control LEW recipients received a vascularized skin allograft alone, without the vascularized bone component, from both fully allogeneic donors.

All LEW recipient received a 7-day immunosuppressive treatment protocol, described in Example 1. Briefly, the treated animal groups received 16 mg/kd/day s.c. of CsA and 250 µg/day i.p. of anti-αβ TCR mAb daily for 7 days. The initial treatments were given at the time of transplantation at the time of clamp release

**Two Genetically Unrelated VSBA Transplants are Accepted Across Fully-Allogeneic MHC Barriers, Producing a Fully Tolerant Trimeric Recipient.** **Figure 9** illustrates a LEW recipient of two genetically unrelated VSBA transplants at day 35 after transplantation. The vascularized bone transplants are not visible, as they are beneath the transplanted skin flaps shown. On the left is a VSBA transplant from a BN donor, showing full skin acceptance by the LEW recipient of this fully allogeneic transplant. On the right is a VSBA transplant from an ACI donor, showing full skin acceptance by the LEW recipient of this fully allogeneic transplant.

**Figures 10A** and **10B** illustrate H&E stained formalin-fixed skin tissues taken from the BN allograft and the ACI allograft, respectively, at day 21 after transplantation, showing preserved dermis and epidermis and no histological signs of rejection. At over 100 days after transplantation (to the present), neither of the skin grafts show any signs of rejection.

**Determination of the Donor Specific Trimerism.** Flow cytometry analysis was performed on PBMC of the LEW recipients at day 21 (**Figure 11**) and day 35 (**Figure 12**) after transplantation of the VSBAS transplants from the BN and ACI donors.

The dot-plot results of the lymphoid cell subpopulations obtained from quadrangle analytical gates demonstrated the presence of double positive RT-1^{a-FITC}/CD4^{-PE}, RT-1^{a-FITC}/CD8^{-PE} and RT-1^{a-FITC}/CD45RA^{-PE} (ACI/LEW) at a level of 8.02%, 4.36% and 0.82%, respectively, of PBMC on day 21 (**Figure 11****,** top horizontal row); and also the presence of double positive RT-1^{n-Cy7}/CD4^{-PE}, RT-1^{n-Cy7}/CD8^{-PE} and RT-1^{n-Cy7}/CD45RA^{-PE} (BN/LEW) at a level of 0.9%, 0.3% and 4.1 %, respectively (**Figure 11****,** bottom horizontal row). The expression of RT-1^{a} (RT-1^{a-FITC}) antigen and RT-1ⁿ (RT-1^{n-Cy7}) antigen (**Figure 11****,** top horizontal row and bottom horizontal row, respectively) on non-CD4, non-CD8, and non-CD45RA positive T cell populations suggests the existence of donor/recipient trimeric residents not evaluated with the PE-conjugated mAb against surface specific antigen.

The dot plot results obtained from the peripheral blood of LEW recipients on day 35 are illustrated in **Figure 12****.** The results demonstrated the presence of double positive RT-1^{a-FITC}/CD4^{-PE}, RT-1^{a-FITC}/CD8^{-PE} and RT-1^{a-FITC}/CD45RA^{-PE} (ACI/LEW) at a level of 7.99%, 4.73% and 0.6%, respectively, of PBMC on day 35 (**Figure 12****,** top horizontal row); and also the presence of double positive RT-1^{n-Cy7}/CD4^{-PE}, RT-1^{n-Cy7}/CD8^{-PE} and RT-1^{n-Cy7}/CD45RA^{-PE} (BN/LEW) at a level of 0,8%, 0.48% and 3.1%, respectively (**Figure 12****,** bottom horizontal row).

These results show that the trimerism obtained in these LEW recipients was stable and correlated with the maintenance of tolerance to both VSBA fully allogeneic allografts.

In contrast, control LEW recipients receiving vascularized skin flaps without vascularized bone transplants showed a transient chimerism (less than 1% to 1%) that declined over time leading to allograft rejection within 40 days from the time of transplantation (data not shown).

Therefore, this example again illustrates the tolerance-inducing and chimerism/trimerism inducing properties of the vascularized bone component of the transplant. It has further been demonstrated that donor-specific cells can be produced in the recipient and can co-exist without rejection in the recipient. No recipient preconditioning is required.

### Example 6

### Immunosuppression of αβ-TCR⁺ Cells in Allograft Recipients Under the Combined CsA/αβ-TCR mAb 35-Day Treatment Protocol Without Administration of Donor Bone Marrow Cells

**Figure 13** illustrates the flow cytometry determination of the efficacy of the immunomodulating therapy of combined CsA/αβ-TCR mAb therapy in the peripheral blood of allograft recipients treated with CsA alone or with the combined CsA/αβ-TCR mAb therapy. The recipients of the combined therapy showed greater than 90% reduction of the αβ-TCR⁺ cells after 21 and 35 days of the combined therapy (p<0.05). Levels were observed to increase, by 16 and 19 fold, by day 50 and 64, respectively, due to re-population of new αβ-TCR⁺ cells. Treatment with CsA alone resulted in a reduction in αβ-TCR⁺ cells of only one-fold observed on day 35 and 1.5-fold on day 50.

### Example 7

### Donor-Specific Chimerism Demonstrated in Allograft Recipients Under the Combined CsA/αβ-TCR mAb 35-Day Treatment Protocol Without Administration of Donor Bone Marrow Cells

Flow cytometric analysis of the donor-specific chimerism revealed double-positive CD4^{PE}/RT-1^{nFITC} (6.7%) and CD8^{PE}/RT-1^{nFITC} (1.2%) T-cell subpopulations in the peripheral blood of hindlimb allograft recipients at 400 days post-transplantation (**Figure 14****, A and B,** respectively). As illustrated in **Figure 15A** and **15B** **,** double-positive donor CD4^{PE}/RT-1^{nFITC} (3.4%) (15A) and CD8^{PE}/RT-1^{nFITC} (12.8%) (15B) T-cell subpopulations were present. In the isograft, allograft, and CsA-alone control groups, no chimeric cells were found in the periphery.

### Example 8

### Donor-Specific Chimerism Demonstrated in Fully Allogeneic Graft Recipients Under the Combined CsA/αβ-TCR mAb 7-Day Treatment Protocol Without Administration of Donor Bone Marrow Cells

Flow cytometric analysis of the donor-specific chimerism using the triple staining technique revealed that, at 120 days post-transplantation, 1.3% of isolated recipient peripheral blood mononuclear cells (PBMC) were CD8⁺ cells of donor origin (**Figure 16** **A**); 7.6% of the isolated recipient PBMC were CD4⁺ cells of donor origin (**16B**); and 16.5% of the recipient PBMC were CD45RA⁺ B cells of donor origin (**16C**).

### Example 9

### Depletion of αβ-TCR⁺ Cells Under a 5-Day, 7-Day, and 21-Day Protocol of Combined CsA/αβ-TCR mAb 7-Day Without Administration of Donor Bone Marrow Cells

As illustrated in **Figure 17****,** the combined use of anti-αβ T cell antibodies and the immunosuppressive agent CsA daily (therapy begun at time of transplantation) for only 21 days, only 7 days, or only 5 days after transplantation successfully depleted αβ TCR⁺ cells in the transplant recipients by virtually 100% at day 7 post-transplantation. The level of depletion in each protocol group remained at greater than 95% to day 35 post-transplantation, creating a 28 day therapeutic window of αβ TCR⁺ cell immunological silence, even when the combined treatment was administered for only 5 to 7 days after transplantation. T cell levels gradually rose until by day 63, they had returned to 50-84% of the pre-transplant level and, by day 90 post-transplantation, had returned to 90-95% of the pre-transplant level, producing clinically observed tolerance of transplanted limbs with no further immunosuppressive therapy.

### Example 10

### Donor-Specific Chimerism Demonstrated in Fully-Allogeneic Graft Recipients Under the Combined CsA/αβ-TCR mAb 5-Day, 7-Day and 21-Day Treatment Protocols Without Administration of Donor Bone Marrow Cells

Flow cytometric analysis of the donor-specific chimerism using the triple staining technique, as illustrated in **Figure 18****,** revealed that at 120 days post-transplantation, using the 5-day protocol, 9.6% of isolated recipient peripheral blood mononuclear cells (PBMC) were CD8⁺ cells of donor origin; 12.3% of the recipient PBMC were CD4⁺ cells of donor origin; and 14.8% of the recipient PBMC were B cells of donor origin. Similarly, using the 7-day protocol, 8.7% of isolated recipient PBMC were CD8⁺ cells of donor origin; 9.4% of the recipient PBMC were CD4⁺ cells of donor origin; and 13.8% of the recipient PBMC were B cells of donor origin. Similarly, using a 21-day protocol in which the combined therapy was begun at the time of transplantation, 6.2% of isolated recipient PBMC were CD8⁺ cells of donor origin; 10.1% of the recipient PBMC were CD4⁺ cells of donor origin; and 11.3% of the recipient PBMC were B cells of donor origin.

### Example 11

### The Level of Chimeric Donor/Recipient Cells in the Peripheral Blood of Allotransplant Recipients Rises Over Time After Transplantation

**Figure 19** illustrates the kinetics of the rise in the level of chimeric donor/recipient cells in the peripheral blood of the combined treatment allotransplant recipients, without the administration of donor bone marrow cells, leading to indefinite graft survival. The Figure illustrates that treated recipients of fully-allogeneic transplants exhibited 5.3% chimeric CD4⁺ cells by about day 14 after transplantation. This level rose to 7.2% by about day 35 after transplantation, and continued to rise to about 14.7% by about day 100 after transplantation. In this rat model, it is evident that a level of chimerism of about 15% by day 100 after transplant ensures indefinite transplant survival.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have elements that do not differ from the literal language of the claims, or if they include equivalent elements with insubstantial differences from the literal language of the claims.

The present invention also relates to the following issues.
1. A method for obtaining mixed donor-recipient chimeric cells from an allograft recipient, comprising the steps of:
   (a) administering to an allograft recipient a short-term immunosuppressive regimen that depletes about 50% to about 99.9% of recipient T cells circulating in peripheral blood of the recipient;
   (b) implanting an allograft from a donor into the recipient;
   (c) allowing the development of mixed donor-recipient chimeric cells in the recipient after completion of the immunosuppressive regimen; and
   (d) harvesting the chimeric cells from the recipient.
2. The method of issue 1, wherein the allograft is selected from the group consisting of composite tissue, solid organs, glands, glandular cells, skin, hematopoietic tissue, lymphoid tissue, tendons, ligaments, muscles, nerve tissue, vascular tissue and combinations thereof.
3. The method of issue 1, wherein the recipient is a rodent.
4. The method of issue 1, wherein the recipient is a mammal.
5. The method of issue 1, wherein the recipient is a human.
6. The method of issue 1, wherein the harvesting step includes the substep of propagating the chimeric cells in culture.
7. The method of issue 1, wherein the harvesting step includes the substep of storing the chimeric cells.
8. The method of issue 1, wherein the step of harvesting step includes the substep of determining expression of chimeric cell surface histocompatability antigens.
9. The method of issue 1, wherein the harvesting step includes the substep of forming a library of mixed donor-recipient chimeric cells from a multiplicity of recipients.
10. The method of issue 1, wherein the implanting step comprises the substep of implanting more than one allograft into the recipient, wherein each donor of the more than one allograft is independently selected from the group consisting of a semi-allogeneic donor, a fully allogeneic donor, and combinations thereof.
11. Mixed donor-recipient chimeric cells harvested from the allograft recipient according to the method of issue 1 or issue 10.
12. A library of mixed donor-recipient chimeric cells harvested from a multiplicity of allograft recipients according to the method of issue 1 or issue 10.
13. An animal model of chimeric recipients produced by the method of issue 1 or issue 10.
14. A method for inducing allograft tolerance and/or mixed donor-recipient chimerism in an allograft recipient, comprising the steps of:
   (a) determining a histocompatibility antigen type of an allograft recipient;
   (b) determining a histocompatibility antigen type of an allograft donor;
   (c) selecting chimeric cells from a library of chimeric cells, wherein the selected chimeric cells represent a best match for the histocompatibility antigen types of both the recipient and the donor;
   (d) administering to an allograft recipient a short-term immunosuppressive regimen that depletes about 50% to about 99.9% of recipient T cells circulating in the peripheral blood of the recipient;
   (e) administering to the recipient a therapeutically effective amount of the selected chimeric cells;
   (f) implanting an allograft from a donor into the recipient, wherein a combination of steps (d) and (e) results in recipient immunological tolerance to the allograft;
   (g) optionally monitoring a level of circulating chimeric cells in the allograft recipient or monitoring the allograft for a visual or histological sign of rejection;
   (h) optionally readministering a therapeutically effective amount of the selected chimeric cells to the recipient if the level of circulating chimeric cells declines to a preselected minimum level or a sign of allograft rejection is observed; and
   (i) optionally readministering a short-term immunosuppressive regimen if the level of circulating chimeric cells declines to a preselected minimum level or a sign of allograft rejection is observed.
15. The method of issue 14, wherein the selected chimeric cells are obtained by cellular fusion of two or more cells having different histocompatibility antigens.
16. The method of issue 14, wherein the preselected minimum level of chimeric cells is a minimum level for preventing onset of allograft rejection.
17. The method of issue 14, wherein the allograft is selected from the group consisting of composite tissue, solid organs, glands, glandular cells, skin, hematopoietic tissue, lymphoid tissue, tendons, ligaments, muscles, nerve tissue, vascular tissue and combinations thereof.
18. The method of issue 14, wherein the recipient is a rodent.
19. The method of issue 14, wherein the recipient is a mammal.
20. The method of issue 14, wherein the recipient is a human.
21. A method for compiling a library of chimeric cells, comprising the steps of:
   (a) harvesting chimeric cells from an allograft recipient according to the method of issue 1 or issue 10 or, alternatively, obtaining chimeric cells by cellular fusion of two or more cells having different histocompatibility antigens;
   (b) determining expression of surface histocompatability antigens of the chimeric cells;
   (c) optionally culturing the harvested chimeric cells to obtain an expanded population of the chimeric cells; and
   (d) storing the chimeric cells.
22. The method of issue 21, wherein the storing step comprises cryogenic storage.

## Claims

1. Use of a therapeutically effective amount of an immunosuppressive agent, and a therapeutically effective amount of anti-αβ T cell receptor antibodies, for the preparation of a pharmaceutical preparation for inducing mixed donor-recipient chimerism in an allograft transplant recipient without pre-conditioning by myeloablation, such that at least 80% of circulating peripheral T-cells are eliminated in said recipient, wherein said anti-αβ T cell receptor antibodies bind the αβ T cell receptor of said circulating peripheral T-cells.

2. The use of claim 1, wherein the recipient comprises a level of donor-recipient chimeric cells of about 5% to about 50% relative to the percentage of recipient circulating peripheral blood mononuclear cells

3. The use of claim 2, wherein the level of donor-recipient chimeric cells is about 10% to about 40% relative to the percentage of recipient circulating peripheral blood mononuclear cells.

4. The use of claim 3, wherein the level of donor-recipient chimeric cells is about 20% to about 30% relative to the percentage of recipient circulating peripheral blood mononuclear cells.

5. The use of claim 1, wherein the allograft is selected from the group consisting of composite tissue, solid organs, glands, glandular cells, skin, cartilage, hematopoietic tissue, lymphoid tissue, tendons, ligaments, muscles, nerve tissue, vascular tissue and combinations thereof.
